# EUROPEAN PATENT SPECIFICATION

(11) **EP 1 706 486 B1**
(45) Date of publication and mention of the grant of the patent: **04.03.2009**
(21) Application number: 05707000.5
(22) Date of filing: 21.01.2005
(51) Int. Cl.: C12N 7/04, A61K 39/12, C12N 15/62, C07K 14/08, C07K 19/00, C12N 15/87, C12N 15/86, C12N 5/10

(54) **CHIMERIC EMPTY CAPSIDS OF THE INFECTIOUS BURSAL DISEASE VIRUS (IBDV), OBTAINMENT PROCESS AND APPLICATIONS**
CHIMÄRISCHE LEERE CAPSIDE DES IBDV (INFECTIOUS BURSAL DISEASE VIRUS), GEWINNUNGSVERFAHREN UND ANWENDUNGEN
CAPSIDES VIDES CHIMERES DU VIRUS DE LA BURSITE INFECTIEUSE (VBI), LEUR PROCEDE D'OBTENTION ET LEURS APPLICATIONS

(30) Priority: 21.01.2004 ES 200400120
(43) Date of publication of application: 04.10.2006
(73) Proprietor: CONSEJO SUPERIOR DE INVESTIGACIONES CIENTIFICAS, 28006 Madrid (ES); Bionostra S.L., 28760 Tres Cantos Madrid (ES)
(72) Inventor: RODRIGUEZ AGUIRRE, José Francisco Centro Nacional, Campus de Cantoblanco 28049 Madrid (ES); RUIZ CASTON, José Centro Nacional, Campus de Cantoblanco 28049 Madrid (ES); GONZALEZ DE LLANO, Maria Dolores Centro Nacional, Campus de Cantoblanco 28049 Madrid (ES); RODRIGUEZ AGUIRRE, Maria Dolores Centro Nacional, Campus de Cantoblanco 28049 Madrid (ES); BLANCO CHAPINAL, Soledad Centro Nacional, Campus de Cantoblanco 28049 Madrid (ES); OÑA BLANCO, Ana Maria Centro Nacional, Campus de Cantoblanco 28049 Madrid (ES); SAUGAR , Irene Cancer Research UK, Herts EN6 3LD (GB); ABAITUA ELUSTONDO, Fernando, Surrey, RH8 0TL (GB); LUQUE BUZO, Daniel Centro Nacional, Campus de Cantoblanco 28049 Madrid (ES); RODRIGUEZ FERNANDEZ-ALBA, Juan Ramón, 28006 Tres Cantos - Madrid (ES)
(74) Representative: Gonzalez-Alberto Rodriguez, Natalia
(86) International application number: PCT/EP2005/000695
(87) International publication number: WO 2005/071069

(56) References cited:
- WO-A-02/088339
- US-A- 5 788 970
- CHEVALIER C ET AL: "The maturation process of pVP2 requires assembly of infectious bursal disease virus capsids" JOURNAL OF VIROLOGY, THE AMERICAN SOCIETY FOR MICROBIOLOGY, US, vol. 76, no. 5, March 2002 (2002-03), pages 2384-2392, XP002218366 ISSN: 0022-538X
- HU Y ET AL: "Chimeric infectious bursal disease virus-like particles expressed in insect cells and purified by immobilized metal affinity chromatography" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 63, no. 6, 20 June 1999 (1999-06-20), pages 721-729, XP002190336 ISSN: 0006-3592
- FERNÁNDEZ-ARIAS A ET AL: "Expression of ORF A1 of infectious bursal disease virus results in the formation of virus-like particles" JOURNAL OF GENERAL VIROLOGY, SOCIETY FOR GENERAL MICROBIOLOGY, READING, GB, vol. 79, no. part 5, May 1998 (1998-05), pages 1047-1054, XP002218365 ISSN: 0022-1317 cited in the application
- MARTINEZ-TORRECUADRADA J L ET AL: "Different Architectures in the Assembly of Infectious Bursal Disease Virus Capsid Proteins Expressed in Insect Cells" VIROLOGY, ACADEMIC PRESS,ORLANDO, US, vol. 278, no. 2, 20 December 2000 (2000-12-20), pages 322-331, XP004435746 ISSN: 0042-6822 cited in the application
- MARTINEZ-TORRECUADRADA J L ET AL: "Structure-dependent efficacy of infectious bursal disease virus (IBDV) recombinant vaccines" VACCINE, BUTTERWORTH SCIENTIFIC. GUILDFORD, GB, vol. 21, no. 23, 4 July 2003 (2003-07-04), pages 3342-3350, XP004429746 ISSN: 0264-410X

## Description

### FIELD OF THE INVENTION

The invention is related to the production of chimeric empty particles of the infectious bursal disease virus (IBDV) and their applications.

### BACKGROUND OF THE INVENTION

Viral particles are structures specialized in the packaging and incorporating in vehicles of nucleic acids and proteins. A general feature of viral particles is their excellent ability for the immune response stimulation of the host. These properties make viral particles agents of extraordinary interest for the development both of intracellular delivery systems and for the generation of particulate vaccines. The use of different genetic expression systems has facilitated the production of viral-like particles or empty viral capsids (VLPs) of different types of viruses (US patent 6,458,362 Casal, et al. 2002. Recombinant VP2 parvoviral pseudo-particles encoding CTL or T-helper cell epitopes; US 5,932,426 Baralle, et al. 1999. Molecular presenting system; US 6,602,705 Barnett, et al. 2003 Expression of HIV polypeptides and production of virus-like particles). The genetic manipulation of these expression systems in turn allows the production of VLPs containing heterologous amino acid sequences coming from proteins other than those forming the native viral capsid. These VLPs are generically called heterotypical, recombinant or chimeric VLPs (CVLPs). CVLPs have mainly been used for two purposes: (i) generation of multivalent vaccines by means of immunologically relevant heterologous peptides (Kingsman, A. J., N. R. Burns, G. T. Layton, and S. E. Adams. 1995. Yeast retrotransposon particles as antigen delivery systems. Ann. N. Y. Acad. Sci. 754: 202-213; Lo-Man, R., P. Rueda, C. Sedlik, E. Deriaud, I. Casal, and C. Leclerc. 1998. A recombinant virus-like particle system derived from parvovirus as an efficient antigen carrier to elicit a polarized Thl immune response without adjuvant. Eur. J. Immunol. 28: 1401-1407; Qiu, Z., D. Ou, H. Wu, T. C. Hobma, and S. Gillam. 1994. Expression and characterization of virus-like particles containing rubella virus structural proteins. J. Virol. 68: 4086-4091); and (ii) modification of the tropism by means of insertion of amino acid sequences involved in interactions with receptor-ligand (Schmidt, U., Rudolf, R, and Bömh, G. 2001. Binding of external ligands onto an engineered virus capsid. Prot. Eng. 14: 769-774; Shin, Y.C., and Folk, W.R. 2003. Formation of polyoma virus-like particles with different VP1 molecules that bind the urokinase plasminogen activator receptor. J. Virol. 77: 11491-11498).

CVLPs are generally obtained by means of the expression of the viral protein(s) responsible for the formation of the viral capsid, fused to the region encoding the polypeptide of interest.

The infectious bursal disease virus (IBDV), belonging to the *Birnaviridae* family, infects different bird species and is directly responsible for a severe immunosuppressive disease causing important economic losses in the world poultry industry.

IBDV particles are icosahedral, with T=13 symmetry, they lack an envelope and are formed by a single protein layer. Up until now, the approaches aimed at obtaining an atomic model for IBDV particles have failed. As a result, the structural information available is based on three-dimensional models generated from images obtained by electron cryomicroscopy of the purified virus and of the VLPs. Based on these studies, it has been verified that the outer surface of the particle is formed by a continuous lattice of 260 trimers of the VP2 protein (37 kDa) organized in five different formations. The inner face of the particles contains 200 trimers of the VP3 protein (29 kDa), the latter, independent from one another, are bound to the basal area of the VP2 trimers. It has been suggested that a third polypeptide, VP4 (28 kDa), could also be part of the particles, being located at the base of the pentamers forming the vertices of the icosahedral structure.

The VP2, VP3 and VP4 polypeptides are produced from the proteolytic processing of a polypeptide precursor of a size of 109 kDa. This precursor is auto-catalytically processed, releasing the pVP2 (48 kDa), VP3 and VP4 polypeptides. The VP4 domain, which is located in the central region of the polyprotein, belongs to the Lon protease family and is responsible for the proteolytic cleavage. The pVP2 and VP3 polypeptides are directly responsible for the capsid assembly. The pVP2 product undergoes a last cleavage at its C-terminal end before giving rise to the mature form of the protein, VP2, which is the one found in purified particles (Da Costa, B., Chevalier, C., Henry, C., Huet, J. C., Petit, S., Lepault, J., Boot, H. & Delmas, B. (2002). The capsid of infectious bursal disease virus contains several small peptides arising from the maturation process of pVP2. Journal of Virology 76:2393-2402). This pVP2 processing is necessary for the correct formation of the capsids and requires the presence of VP3, although the responsible protease has not yet been identified (Maraver, A., Oña, A., Abaitua, F., González, D., Clemente, R., Diaz-Ruiz, A., Caston, J. R., Pazos, F. & Rodriguez, J. F. (2003). The oligomerization domain of VP3, the scaffolding protein of infectious bursal disease virus, plays a critical role for capsid formation. Journal of Virology 77:6438-49).

In general terms, morphogenesis is a vital process for the viral cycle requiring successive steps associated to modifications in the polypeptide precursors. As a result, viruses have developed strategies allowing the sequential and correct interaction between each one of their components. One of these strategies, frequently used by icosahedral viruses, is the use of polypeptides coming from a single polyprotein as the base of their structural components. In these cases, the suitable proteolytic processing of said polyprotein plays a crucial role in the assembly process.

This concept for the assembly of IBDV capsids has been demonstrated in earlier work (Fernández-Arias, A., Risco, C., Martínez, S., Albar, J. P. & Rodriguez, J. F. (1998). Expression of ORF A1 of infectious bursal disease virus results in the formation of virus-like particles. Journal of General Virology 79, 1047-1054). Expression of the gene encoding for the IBDV polyprotein in eukaryotic cells gives rise to the formation of VLPs that are completely morphologically and biochemically indistinguishable from the IBDV virions. It has also been shown that the assembly of the capsids requires only the synthesis and correct processing of the viral polyprotein and is independent of the presence of the viral genome or of other proteins encoded by the viral genome, such as VP5 and VP1.

The results obtained to date from the IBDV gene expression in different recombinant systems has allowed concluding that: i) the assembly process is independent of the presence of genetic material of the virus, ii) only the polypeptides encoded by the polyprotein gene are necessary for the assembly, and iii) the assembly requires a coordinated interaction between the pVP2 and VP3 polypeptides.

However, it must be indicated that it is not known if the VP2/VP3 interaction is established between VP2 and VP3 domains of the polyprotein precursor when it has not yet undergone modifications, or on the contrary, if this interaction occurs after the processing of the precursor. Furthermore, current information does not exclude the possibility that VP4 could play a relevant role in the morphogenesis of the viral capsid. In fact, IBDV VLPs formed by assembly of the IBDV VP2, VP3 and VP4 proteins have been disclosed (US 6,528,063, US 5,788,970 and JP 5194597).

The work developed by the same inventors has enabled establishing systems for obtaining IBDV VLPs using different eukaryotic expression vectors. These vectors have been used for IBDV polyprotein expression in the absence or presence of the viral VP1 RNA polymerase. The biochemical characterization of purified VLPs demonstrates that they contain pVP2, VP2 and VP3 proteins when only the viral polyprotein is expressed, and the pVP2, VP2, VP3 and VP1 proteins when the simultaneous expression of the polyprotein and viral RNA polymerase is carried out (Fernández-Arias, A, Risco, C., Martínez, S., Albar, J. P. & Rodríguez, J. F. (1998). Expression of ORF A1 of infectious bursal disease virus results in the formation of virus-like particles. Journal of General Virology 79: 1047-1054; Martínez-Torrecuadra, J. I., Castón, J. R., Castro, M., Carrascosa, J. L., Rodríguez, J. F. & Casal, J. L (2000). Different architectures in the assembly of infectious bursal disease virus capsid proteins expressed in insect cells. Virology 278: 322-331; Maraver, A., *et al.,* (2003) cited *supra*; Lombardo, E., Maraver, A., Castón, J. R., Rivera, J., Fernández-Arias, A., Serrano, A., Carrascosa, J. L. & Rodríguez,, J. F. (1999). VP1, the putative RNA-dependent RNA polymerase of infectious bursal disease virus, forms complexes with the capsid protein VP3, leading to efficient encapsidation into virus-like particles. Journal of Virology 73: 6973-6983).

On the other hand, patent application WO 02/088339 discloses IBDV viral-like particles formed by assembly of chimeric proteins comprising the IBDV polyprotein bound at its carboxyl terminal end to a polypeptide.

Additionally, virus like particles formed when the GFP-sequence is fused to the C-terminal domain of the pVP2-VP4-VP3 polyprotein, using a baculovirus expression system, have been described (Chevalier C et al., (2002). The maturation process of pVP2 requires assembly of infectious bursal disease virus capsids. Journal of Virology, The American Society, for Microbiology US 76.5: 2384-2392).

However, CVLPs solely based on IBDV pVP2 and VP3, the latter VP3 protein being fused to a polypeptide of interest, or their potential as vehicles of products of interest, have not been previously disclosed.

### SUMMARY OF THE INVENTION

The invention is faced with the problem of providing new tools for incorporating in vectors or vehicles products of interest, such as molecules with biological activity, for example drugs, polypeptides, proteins, nucleic acids, etc.

The solution provided by this invention is based on it being possible to generated, based on the simultaneous expression of the IBDV pVP2 and VP3 proteins, the latter genetically modified to include a nucleotide sequence encoding for a heterologous polypeptide comprising a polypeptide of interest, IBDV chimeric empty capsids (CVLPs). The resulting CVLPs are formed by assembly of (i) IBDV pVP2 proteins and (ii) fusion proteins comprising a region A constituted by the IBDV VP3 protein bound to a legion B constituted by a heterologous polypeptide comprising a polypeptide of interest, wherein said region B is bound to the amino- end of said IBDV VP3 proteins. These CVLPs can be used for therapeutic, preventive or diagnostic purposes, etc., for example in the manufacture of gene therapy vectors or vaccines.

The inventors had previously found that when IBDV VPX (pVP2) and VP3 proteins are expressed from independent genes, empty IBDV particles (VLPs) are formed. These VLPs are structurally identical to those obtained by means of expression of the ORF corresponding to the IBDV polyprotein. As part of the development of new vaccination strategies, the possibility of using this IBDV VLP production strategy for obtaining CVLPs which contained heterologous amino acid sequences, corresponding to peptides of interest, such as a histidine tag (Example 1), GFP (Example 2) and finally peptides involved in immune response induction (Example 3), was analyzed. As is demonstrated, the fusion of heterologous sequences in these constructs is not an obstacle for the formation of CVLPs.

As a study model of peptide-transporting CVLPs involved in an immune response, the possibility of obtaining CVLPs which had the sequence corresponding to the CD8 epitope (E-CD8) of the malaria CS protein *(Plasmodium yoelii)* was approached. This epitope is responsible for the CD8-specific cellular immune response induction against this pathogen, which can be quantified by means of the ELISPOT technique in splenocyte cultures from BALB/c mice (Example 3).

In summary, the obtained results clearly show that: (i) the expression system used allows obtaining IBDV CVLPs containing heterologous amino acid sequences; and (ii) immunization with said IBDV CVLPs induces a specific immune response to the heterologous amino acid sequence present in the CVLPs.

Therefore, an aspect of the present invention is related to an IBDV chimeric empty capsid characterized in that it is constituted by assembly of (i) IBDV pVP2 proteins and (ii) fusion proteins comprising a region A constituted by the IBDV VP3 protein bound to a region B constituted by a heterologous polypeptide comprising a polypeptide of interest.

A further aspect of this invention is related to a process for producing said IBDV CVLPs provided by this invention, based on the gene coexpression of said IBDV pVP2 and fusion proteins as two independent genes.

The nucleic acids, gene constructs, expression systems and host cells developed for implementing said process of producing said IBDV CVLPs, as well as their use for the production of said IBDV CVLPs, constitute further aspects of the present invention.

Said IBDV CVLPs have the ability to incorporate in vectors or vehicles products of interest such as molecules with biological activity, for example, polypeptides, proteins, nucleic acids, etc. In a particular embodiment, said IBDV CVLPs can internally incorporate in vehicles antigens and immune response inducers in animals or humans to whom it is supplied, whereby they can be used in the manufacture of vaccines against human and animal diseases caused by viruses, bacteria, parasites or any other type of microorganism or against tumor diseases. In another particular embodiment, said IBDV CVLPs are used in the manufacture of gene therapy vectors.

Therefore, in a further aspect, the present invention is related to the use of said IBDV CVLPs in the manufacture of medicaments, such as vaccines and gene therapy vectors. Said vaccines and vectors constitute further aspects of the present invention.

### BRIEF DESCRIPTION OF THE FIGURES

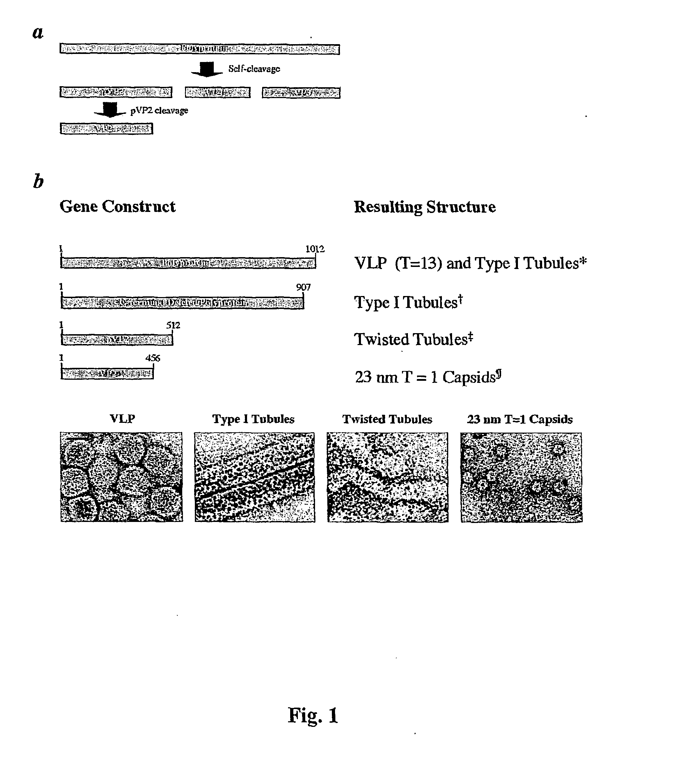

**Figure 1****.** (*a*) The diagram schematizes the proteolytic processing steps necessary for the formation of mature VP2 and VP3 capsid proteins from the polyprotein precursor. (b) The diagram reflects different genetic constructs derived from the IBDV polyprotein described up until now, as well as the structures produced by means of its expression in different heterologous systems. The numbers indicate the position corresponding to the first and last amino acid residue of the polyprotein present in each one of the constructs. The lower portion of the figure shows images obtained by means of transmission electron microscopy of the structures obtained by means of expression of the different constructs. The bar corresponds to 50 nm. The data has been taken from the following literature references: Fernández-Arias *et al.,* (1998), cited *supra;* Maraver *et al.,* (2003), cited *supra;* Martinez-Torrecuadrada *et al.,* (2000), cited *supra;* Castón et al., 2001. C terminus of infectious bursal disease virus major capsid protein VP2 is involved in definition of the t number for capsid assembly. Journal of Virology 75, 10815-10828.

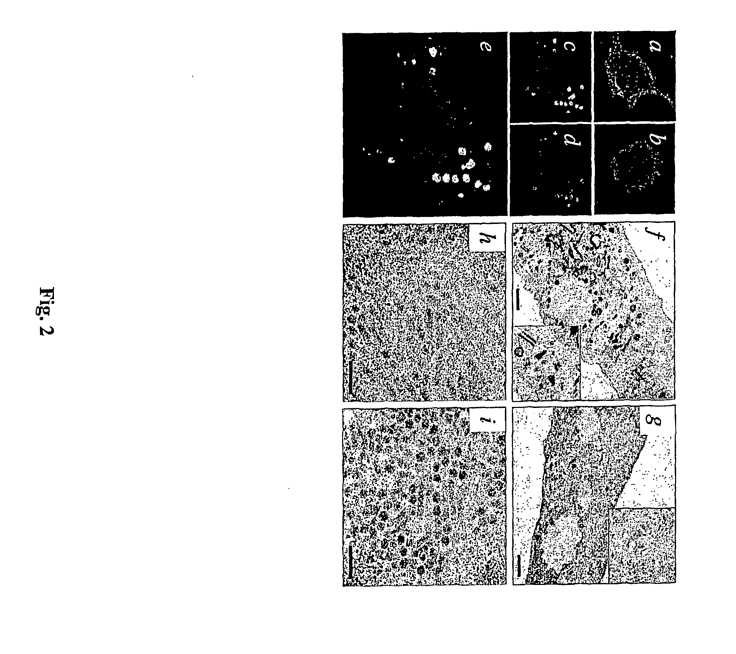

**Figure 2****. Microscopic analysis of H5 insect cells coexpressing pVP2 and VP3.** The pVP2 and VP3 protein subcellular distribution was analyzed by means of confocal immunomicroscopy. Cells infected with the FB/pVP2 (a), FB/VP3 (b), or FBD/pVP2-VP3 (c-e) rBVs were incubated with rabbit anti-pVP2 serum and rat anti-VP3 serum. Then the cells were incubated with goat anti-rabbit IgG serum coupled to Alexa 488 (red) and goat anti-rat IgG serum coupled to Alexa 594 (green). The cores were stained with To-Pro 3 (blue). (e) Overlaying of the images shown in panels (c) and (d). Electron microscopy images corresponding to sections of H5 cells infected with different genetic constructs derived from the IBDV polyprotein. (f) Low-magnification image of an H5 cell infected with a parental Fb virus. The insert corresponds to an enlarged detail of the area indicated by the box. (g) Low-magnification image of an H5 cell infected with the FBD/pVPX-VP3 virus. The insert corresponds to an enlarged detail of the area indicated by the box. (h) High-magnification image of an H5 cell infected with the FBD/pVPX-VP3 virus showing the formation of IBDV structures in detail. (i) High-magnification image of a BSC1 cell infected with the VTLacOI/POLY recombinant vaccine virus showing structures similar to those detected in panel (h). The bars indicate 600 nm (panels f and g) and 200 nm (panels h and i).

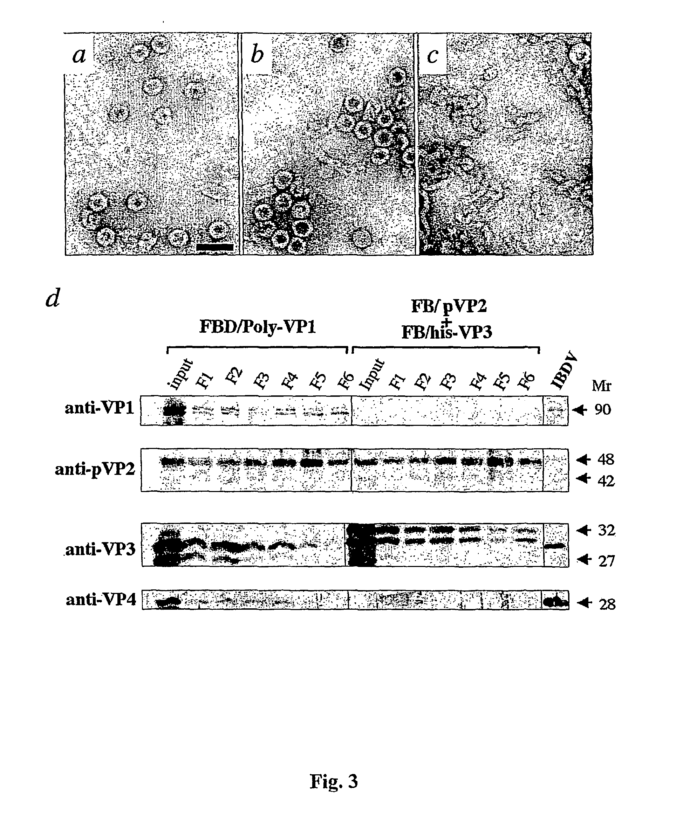

**Figure 3****. Structural and biochemical characterization of the structures derived from IBDV produced in insect cells coinfected with the FB/pVP2 + FB/his-VP3 recombinant baculoviruses (rBV).** Cells coinfected with FB/pVP2 and FB/his-VP3 rBVs, or infected with the FBD/Poly-VP1 or FB/pVP2 virus, were used to purify structures derived from IBDV by means of centrifugation on sucrose gradients. Panels *(a), (b),* and *(c)* show transmission electron microscopy images corresponding to fraction 4 of the gradients obtained from infections with FBD/Poly-VP1, FB/pVP2+FB/his-VP3, and FB/pVP2, respectively. Panel (d) shows the results of a Western blot analysis of the sucrose gradients corresponding to the cultures infected with FBD/Poly-VP1 and FB/pVP2+FB/his-VP3, respectively. The total extracts (input) and the different fractions of the sucrose gradients (fraction F1 corresponds to the bottom of the gradient) were analyzed by means of Western blot using specific sera against the IBDV VP1, pVP2, VP3, and VP4 proteins, respectively. The molecular mass of the immunoreactive polypeptides is indicated in kDa.

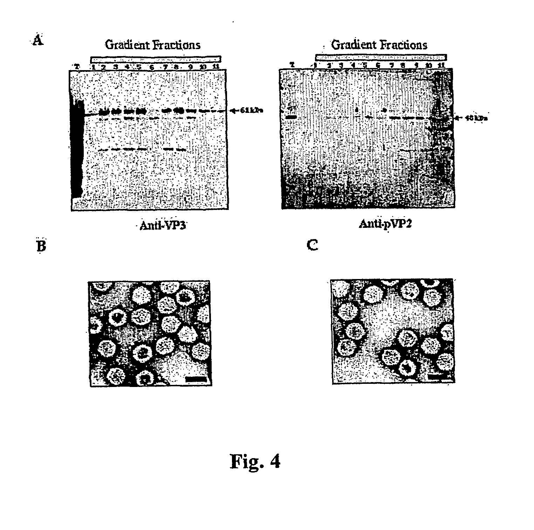

**Figure 4****. Biochemical and structural characterization of IBDV VLPs produced in *S. cerevisiae* transformed with the plasmid pESCURA/pVP2-VP3-GFP.** A *S. cerevisiae* culture transformed with the plasmid pESCURA/pVP2-VP3-GFP was grown at 30°C in a medium supplemented with the inducer galactose. At 18 hours, the culture was harvested and centrifuged. The resulting sediment was processed by means of fractioning in a 25-50% linear sucrose gradient. A) Biochemical analysis of samples corresponding to the sediment before fractioning (T) as well as the different fractions of the sucrose gradient. The samples were analyzed by means of SDS-PAGE and Western blot using specific antibodies against VP3 (anti-VP3) and pVP2 (anti-pVP2) proteins. The arrows indicate the positions of the immunoreactive bands corresponding to the VP3-GFP (61 kDa) and pVP2 (48 kDa) proteins, respectively. B) The structural analysis of the obtained samples was carried out by means of TEM. The image corresponds to a micrography obtained from an aliquot corresponding to the mixture of fractions 7, 8 and 9 of the sucrose gradient. The sample was stained with uranyl acetate and observed by means of TEM. The bar corresponds to 65 nm. C) VLPs sample obtained by means of the IBDV polyprotein expression in mammal cells by means of infection with the VT7/Poly recombinant vaccine virus (Fernández-Arias *et al.,* (1998), cited *supra*). The bar corresponds to 65 mn.

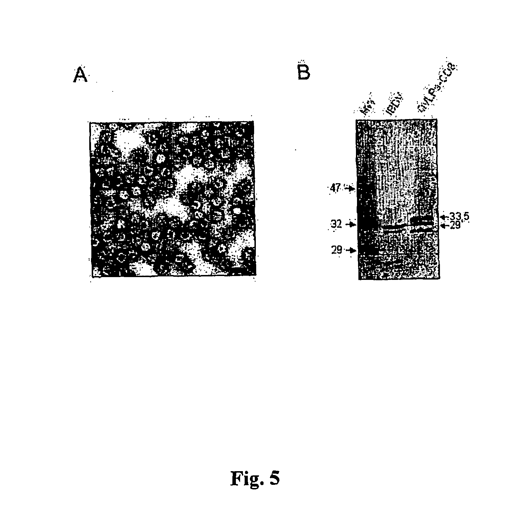

**Figure 5****. Structural and biochemical characterization of QVLPs-CD8.** Panel A shows a TEM image of a sample stained with uranyl acetate corresponding to fraction 4 of a sucrose gradient used for the purification of structures carried out on an insect cell extract coinfected with the FB/pVP2 and PF/his-CD8-VP3 rBVs. The bar indicates 100 nm. Panel B shows the SDS-PAGE and Western blot analyses carried out with an antibody against VP3 protein, of a sample corresponding to fraction 4 (QVLPs-CD8) of a sucrose gradient used for the purification of structures carried out on an insect cell extract co-infected with the FB/pVP2 and PF/his-CD8-VP3 rBVs (see panel A). A sample of purified IBDV virus (IBDV) was used as a control. The sizes of the molecular mass (MW) markers, as well as the molecular mass estimated for the VP3 and his-CD8-VP3 proteins, were indicated.

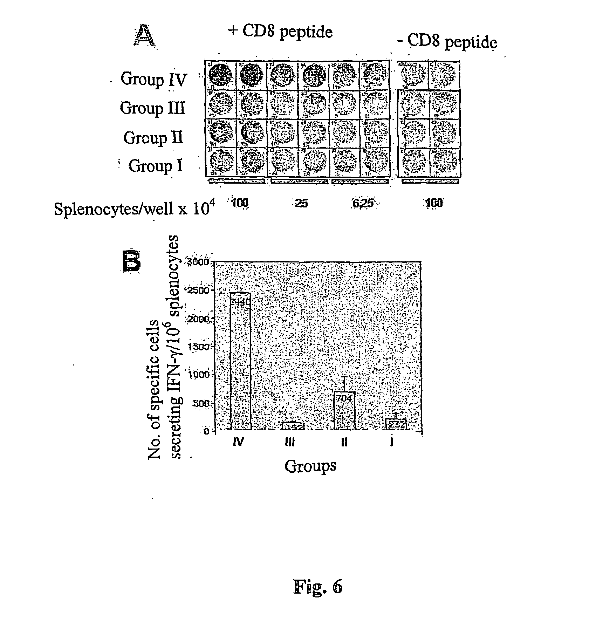

**Figure 6****.- Enhancing effect of the specific anti-malaria CD8 cellular immune response by means of immunization with IBDV CVLPs containing the *Plasmodium yoelii* CD8 epitope.** Groups of 4 mice from the BALB/c strain were intraperitoneally inoculated with 50 µg/mouse of QVLPs-CD8 (group IV) or non-chimeric VLPs (group IIII). A group was inoculated with VVpJRCS (10⁷ pfu/mouse), a recombinant virus expression the whole *Plasmodium yoelii* CHITOSAN protein (group II) as a control. 15 days later, the mice of all the groups were intraperitoneally immunized with WpJRCS (10⁷ pfu/mouse). One of the groups received at that time a single dose of the viral vector (group I). 15 days after the second immunization, the animals were sacrificed, the spleen was removed and the ELISPOT was carried out against the malaria CD8 peptide. Panel A shows the image of the ELISPOT wells carried out with different concentrations of splenocytes obtained from the mice belonging to each one of the groups after their incubation in the presence (+CD8 peptide) or absence (-CD8 peptide) of the CD8 peptide. Panel B shows a graph of the results obtained as a number of specific IFN-γ/10⁶ splenocyte secreting cells.

### DETAILED DESCRIPTION OF THE INVENTION

In a first aspect, the invention provides an chimeric empty capsid of the infectious bursal disease virus (IBDV), hereinafter CVLP of the invention, characterized in that it is constituted by assembly of (i) IBDV pVP2 proteins and (ii) fusion proteins comprising a region. A constituted by the IBDV VP3 protein bound to a region B constituted by a heterologous polypeptide comprising a polypeptide of interest.

The term "IBDV", as it is used in the present invention, refers to the different IBDV strains belonging to any of the known serotypes (1 or 2) [by way of illustration, see the review carried out by van den Berg TP, Eterradossi N, Toquin D, Meulemans G. en Rev Sci Tech 2000 19: 509-43] and the terms "IBDV pVP2 protein" and "IBDV VP3 protein" refer to the different forms of the pVP2 and VP3 proteins representative of any of the mentioned IBDV strains [NCBI protein databank], according to the definition made by Sánchez and Rodríguez (1999) (Sánchez AB, Rodríguez JF. Proteolytic processing in infection bursal disease virus: identification of the polyprotein cleavage sites by site-directed mutagenesis. Virology. 1999 Sep 15; 262(1):190-199), as well as proteins substantially homologous to said IBDV pVP2 and VP3 proteins, i.e. proteins the amino acid sequences of which have a degree of identity regarding said IBDV pVP2 and VP3 proteins of at least 60%, preferably of at least 80%, more preferably of at least 90% and even more preferably of at least 95%.

The BBDV pVP2 protein present in the CVLP of the invention can be any pVP2 protein representative of any IBDV strain, for example, the full-length pVP2 protein of IBDV *Soroa* strain [NCBI, access number AAD30136].

The fusion protein present in the CVLP of the invention comprises a region A constituted by the IBDV VP3 protein bound to a region B constituted by a heterologous polypeptide comprising a polypeptide of interest In a particular embodiment, said region B is bound to the amino-terminal region of said IBDV VP3 protein.

The IBDV VP3 protein, constituting region A of said fusion protein, can be any VP3 protein representative of any IBDV strain, for example, the full-length VP3 protein of IBDV Soroa strain [NCBI, access number AAD30136].

Region B present in said fusion protein is constituted by a heterologous polypeptide comprising a polypeptide of interest. As it is used in the present invention, the term "heterologous polypeptide" refers to a polypeptide not belonging to the native IBDV capsid. The size of the polypeptide of interest can vary within a broad interval, from a few amino acids up to hundreds of amino acids. Said polypeptide of interest can be virtually any polypeptide, regardless of its origin (eukaryotic, prokaryotic, viral, etc.), susceptible to being expressed in a recombinant manner. However in a particular embodiment said polypeptide of interest is a polypeptide useful in vaccination, therapy or diagnosis, such as an epitope or determining antigen capable of inducing an immune response in animals and humans against diseases caused by viruses, bacteria, parasites or any other type of microorganism, or against tumor diseases.

In a particular embodiment, said region B comprises a single polypeptide of interest. However, in another particular embodiment, said region B comprises two or more polypeptides of interest, equal or different, which can be forming tandems.

In a particular embodiment, said fusion protein comprises a region A bound to a single region B. In this case, said region B can be bound to the amino-teminal region of VP3, or, present in region A. As previously mentioned region B can contain one or more polypeptides of interest. In a particular embodiment, said region B contains a single polypeptide of interest, whereas in another particular embodiment, said region B comprises two or more different polypeptides of interest.

In another particular embodiment, said fusion protein comprises a region A bound to two regions B, one of them bound to the amino-terminal region of VP3 present in region A and the other one to the carboxy-terminal region of VP3 present in region A. Said regions B can be equal or different, and each one of them can contain one or more polypeptides of interest, which can be equal to or different from one another. In a specific embodiment, the fusion protein comprises a region A bound to a first region B containing a first polypeptide of interest (B1) and a second region B containing a second polypeptide of interest (B2). Said polypeptides of interest (B1) and (B2) can be equal or different. In a specific embodiment, said polypeptides of interest (B1) and (B2) are different from one another.

Generally, region A, (constituted by the IBDV VP3 protein) is not bound directly to said region B (constituted by the heterologous polypeptide comprising a polypeptide of interest), but rather through a linker polypeptide between said regions A and B. Therefore, if desired the fusion protein of the invention can further captain a linker polypeptide located between said regions A and B. Advantageously, said linker polypeptide is a peptide with structural flexibility, preferably a polypeptide giving rise to a non-structured domain able to induce an immune response or not. By way of illustration, said flexible peptide can contain repetitions of amino acid residues, particular Gly and Ser residues, or any other suitable repetition of amino acid residues.

The CVLPs of the invention can be obtained by means of the simultaneous expression of said IBDV pVP2 proteins and said fusion protein comprising said regions A and B, in suitable host cells. Said suitable host cells are cells containing the encoding nucleotide sequence of said fusion protein comprising regions A and B, and the encoding nucleotide sequence of the IBDV pVP2 protein, either in a single gene construct or in two gene constructs. In a particular embodiment, said suitable host cells are cells that are transformed, transfected or infected with a suitable expression system, such as an expression system comprising a gens construct wherein said gene construct comprises the nucleotide sequence encoding for said fusion protein comprising regions A and B, and the nucleotide sequence encoding for the IBDV pVP2 protein, or else alternatively with an expression system comprising a first gene construct comprising the nucleotide sequence encoding for said fusion protein comprising regions A and B, and a second gene construct comprising the nucleotide sequence encoding for the IBDV pVP2 proteins.

Therefore, in another thy invention provides a nucleic acid, the nucleotide sequence of which comprises the nucleotide sequence encoding for said fusion protein forming part of the CVLP of the invention and comprising a region A constituted by the IBDV VP3 protein bound to a region B constituted by a heterologous polypeptide comprising a polypeptide of interest, wherein said regions B bound to the atnino-tetminal region of said IBDV VP3 protein. Optionally, the nucleic acid provided by this invention can contain the nucleotide sequence encoding for IBDV pVP2 if desired. More specifically, in a particular embodiment, the nucleic acid sequence provided by this invention comprises (i) a nucleotide sequence comprising the open reading frame or encoding region corresponding to the IBDV VP3 protein and (ii) a nucleotide sequence comprising the open reading frame or encoding region of one or more heterologous polypeptides comprising one or more polypeptides of interest, and optionally if desired, (iii) a nucleotide sequence comprising the open reading frame or encoding regions corresponding to the IBDV pVP2 protein.

In another particular embodiment, the nucleic acid sequence provided by this invention comprises (i) a nucleotide sequence comprising the open reading frame or encoding region corresponding to the IBDV VP3 protein, (ii) a first nucleotide sequence composing the open reading frame or encoding region of one or more heterologous polypeptides comprising the open reading frame or encoding region of one or more heterologous polypeptide comprising one or more polypeptide of interest (ii') a second nucleotide sequence comprising the open reading frame or encoding region of one or more heterologous polypeptides comprising one or more polypeptides of interest, wherein said nucleotide sequence can be equal to or different from each first nucleotide sequence, and optionally if desired, (iii) a nucleotide sequence comprising the open reading frame or encoding region corresponding to the IBDV pVP2 protein. In this case, one of said first or second nucleotide sequence is operatively bound to 5' end of the nucleotide sequence comprising the open reading frame or encoding region corresponding to the IBDV VP3 protein and the other one is operatively bound to the 3' end of the nucleotide sequence comprising the open reading frame or encoding region corresponding to the IBDV VP3 protein.

As it is used in this description, the term "open reading frame corresponding to the pVP2 protein" or "open reading frame corresponding to the IBDV VP3 proteins" includes, apart from the nucleotide sequences of said open reading frames, other open reading frames analogous to the same encoding frames of the IBDV pVP2 and VP3 proteins. Likewise, the term "open reading frame of one or more heterologous polypeptides comprising one or more polypeptides", includes any encoding nucleotide sequence of said heterologous polypeptide(s) comprising one or more polypeptides of interest. As it is used herein, the term "analogous" intends to include any nucleotide sequence which can be isolated or constructed on the base of the encoding nucleotide sequence of IBDV pVP2 and VP3, for example by means of the introduction of conservative or non-conservative nucleotide replacements, including the insertion of one or more nucleotides, the addition of one or more nucleotides at any of the ends of the molecule, or the deletion of one or more nucleotides at any end or inside of the sequence. Generally, a nucleotide sequence analogous to another nucleotide sequence is substantially homologous to said nucleotide sequence. In the sense used in this description, the expression "substantially homologous" means that at the nucleotide level, the nucleotide sequences in question have a degree of identity of at least 60%, preferably of at least 80%, more preferably of at least 90%, and even more preferably of at least 95%.

In another aspect, the invention provides a gene construct comprising a nucleic acid provided by this invention, i.e. a nucleic acid the nucleotide sequence of which comprises the nucleotide sequence encoding for said fusion protein comprising regions A and B, and optionally the nucleotide sequence encoding for said IBDV pVP2 protein. More specifically, in a particular embodiment, the gene construct provided by this invention comprises a nucleotide sequence comprising (i) a nucleotide sequence comprising the open reading frame or encoding region corresponding to the IBDV VP3 protein and (ii) a nucleotide sequence comprising the open reading frame or encoding region of one or more heterologous polypeptides comprising one or more polypeptides of interest, and optionally if desired, (iii) a nucleotide sequence comprising the open reading frame or encoding region corresponding to the IBDV pVP2 protein. In another particular embodiment, the gene construct provided by this invention comprises a nucleotide sequence comprising (i) a nucleotide sequence comprising (i) a nucleotide sequence comprising the open reading frame or encoding region corresponding to the IBDV VP3 protein, (ii) a first nucleotide sequence comprising the open reading frame or encoding region of one or more heterologous polypeptides comprising one or more polypeptides of interest, (ii') a second nucleotide sequence comprising the open reading frame or encoding region of one or more heterologous polypeptides comprising one or more polypeptides of interest, wherein said second nucleotide sequence can be equal to or different from said first nucleotide sequence, and optionally if desired (iii) a nucleotide sequence comprising the open reading frame or encoding region corresponding to the IBDV pVP2 protein. In this case, one of said first or second nucleotide sequences is operatively bound to the 5' end of the nucleotide sequence comprising the open reading frame or encoding region corresponding to the IBDV VP3 protein, and the other one is operatively bound to the 3' end of the nucleotide sequence comprising the open reading frame or encoding region corresponding to the IBDV VP3 protein.

In another aspect, the invention provides an expression vector or system selected from:
a) an expression system comprising a gene construct provided by this invention, operatively bound to transcription, and optionally translation, control elements, wherein said gene construct comprises the nucleotide sequence encoding for said fusion protein comprising regions A and B and the nucleotide sequence encoding for the IBDV pVP2 protein; and
b) an expression system comprising a first gene construct provided by this invention, operatively bound to transcription, and optionally translation, control elements, wherein said first construct comprises the nucleotide sequence encoding for said fusion protein comprising regions A and B, and a second gene construct operatively bound to transcription, and optionally translation, control elements, comprising the nucleotide sequence encoding for the IBDV pVP2 protein.

In a particular embodiment, the expression system provided by this invention comprises a gene construct comprising (i) a nucleotide sequence comprising the open reading frame or encoding region corresponding to the IBDV VP3, (ii) a nucleotide sequence comprising the open reading frame or encoding region of one or more heterologous polypeptides comprising one or more polypeptides of interest, and (iii) a nucleotide sequence comprising the open reading frame or encoding region corresponding to the IBDV pVP2 protein, wherein said gene construct is operatively bound to transcription, and optionally translation, control elements.

In another particular embodiment, the expression system provided by this invention comprises a first gene construct, operatively bound to transcription, and optionally translation, control elements, said first gene construct comprising (i) a nucleotide sequence comprising the open reading frame or encoding region corresponding to the IBDV VP3 protein, and (ii) a nucleotide sequence comprising the open reading frame or encoding region of one or more heterologous polypeptides comprising one or more polypeptides of interest, and a second gene construct, operatively bound to transcription, and optionally translation, control elements, said second gene construct comprising a nucleotide sequence comprising the open reading frame or encoding region corresponding to the IBDV pVP2 protein

The transcription, and optionally translation, control elements present in the expression system provided by this invention include promoters, directing the transcription of the nucleotide sequences of interest (pVP2, VP3 and heterologous polypeptide) to which it is operatively linked, and other sequences necessary or suitable for the transcription and its suitable regulation in time and place, for example, start and end signals, cleavage sites, polyadenylation signal, replication origin, transcriptional activators (enhancers), transcriptional silencers (silencers), etc.

Virtually any suitable expression system or vector can be used in the generation of the expression system provided by this invention. By way of illustration, said suitable expression or vector systems can be selected, according to the conditions and needs of each specific case, from plasmids, bacmids, yeast artificial chromosomes (YACs), bacteria artificial chromosomes (BACs), bacteriophage P1-based artificial chromosomes (PACs), cosmids, or viruses, which can further have a heterologous replication origin, for example, bacterial or of yeast, so that it may be amplified in bacteria or yeasts, as well as a marker usable for selecting the transfected cells different from the gene or genes of interest. These expression systems or vectors can be obtained by conventional methods known by persons skilled in the art [Sambrook, J., Fritsch, E.F., and Maniatis, T. (1989). Molecular cloning: a laboratory manual, 2nd ed. Cold Spring Harbor Laboratory] and form part of the present invention. In a particular embodiment, said expression or vector system is a plasmid, such as a plasmid suitable for transforming yeasts, for example, the plasmid called pESCURA/pVP2-VP3-OFP (Example 2), or a virus, such as a recombinant baculovirus (rBV), for example, the rBV called FBD/pVP2-his-VP3 (Example 1.2), simultaneously expressing both proteins (IBDV pVP2 and his-VP3) in insect cells during the replication cycle, or the rBVs called FB/pVP2 and FB/his-VP3 (Example 1.1) expressing the IBDV pVP2 and his-VP3 proteins, respectively, when coinfecting insect cells, obtaining IBDV CVLPs with the six histidine (6 his) heterologous polypeptide, or the rBVs called FB/pVP2 and FB/his-CD8-VP3 (Example 3) expressing IBDV pVP2 proteins and his-CD8-VP3, respectively, when co-infecting insect cells, forming the capsids called CD8-CVLPs.

In another aspect, the invention provides a host cell containing the encoding nucleotide sequence of said fusion protein comprising regions A and B, and the encoding nucleotide sequence of the IBDV pVP2 protein, either in a single gene construct or in two different gene constructs. In a particular embodiment, said host cell is a host cell that is transformed, transfected or infected with (i) an expression system provided by this invention comprising either a gene construct wherein said gene construct comprises the nucleotide sequence encoding for said IBDV pVP2 protein and the nucleotide sequence encoding for said fusion protein comprising regions A and B, and the encoding nucleotide sequence of the IBDV pVP2 protein, or else alternatively with (ii) an expression system comprising a first construct comprising the nucleotide sequence encoding for said fusion protein comprising regions A and B, and second gene construct comprising the nucleotide sequence encoding for said IBDV pVP2 protein.

In a particular embodiment, the host cell provided by this invention is a host cell that is transformed, transfected or infected with an expression system comprising a gene construct comprising (i) a nucleotide sequence comprising the open reading frame or encoding region corresponding to the IBDV VP3 protein, (ii) a nucleotide sequence comprising the open reading frame or encoding region of a heterologous polypeptide comprising a polypeptide of interest, and (iii) a nucleotide sequence comprising the open reading frame or encoding region corresponding to the IBDV pVP2 protein, wherein said gene construct is operatively bound to transcription, and optionally translation, control elements.

In another particular embodiment, the host cell provided by this invention is a host cell that is transformed, transfected or infected with (a) a first gene construct, operatively bound to transcription, and optionally translation, control elements, said first gene construct comprising (i) a nucleotide sequence comprising the open reading frame or encoding region corresponding to the IBDV VP3 protein, and (ii) a nucleotide sequence comprising the open reading frame or encoding region of a heterologous polypeptide comprising a polypeptide of interest, and with (b) a second gene construct, operatively bound to transcription, and optionally translation, control elements, said second gene construct comprising a nucleotide sequence comprising the open reading frame or encoding region corresponding to the IBDV pVP2 protein.

Virtually any host cell susceptible to being transformed, transfected or infected by an expression system provided by this invention can be used, for example, mammal cells, bird cells, insect cells, yeasts, etc; however, in a particular embodiment, said host cell is selected from yeasts and insect cells. Yeasts are suitable due to the simplicity and production cost. Insect cells are suitable when the expression system comprises one or two recombinant baculoviruses (rBV). The use of rBV is advantageous due to biosafety issues related to the host range of the baculoviruses, incapable of replicating in other cell types which are not insect cells.

In a particular embodiment, the invention provides a host cell, such as a yeast, for example, *Saccharomyces cerevisiae, Saccharomyces pombe,* etc., transformed with an expression system, such as a plasmid or an expression vector, comprising a gene construct provided by this invention comprising the nucleotide sequence encoding for said fusion protein comprising regions A and B, and the nucleotide sequence encoding for the IBDV pVP2 protein.

In another particular embodiment, the invention provides a host cell, such as an insect cell, infected with an expression system, such as a recombinant baculovirus, comprising a gene construct provided by this invention comprising the nucleotide sequence encoding for said fusion protein comprising regions A and B, and the nucleotide sequence encoding for the IBDV pVP2 protein.

In another particular embodiment, the invention provides a host cell, such as an insect cell, coinfected with an expression system comprising a first recombinant baculovirus comprising a gene construct provided by this invention comprising the nucleotide sequence encoding for said fusion protein comprising regions A and B, and with a second recombinant baculovirus comprising a gene construct provided by this invention comprising the nucleotide sequence encoding for the IBDV pVP2 protein.

In another aspect, the invention provides a process for the production of CVLPs of the invention comprising culturing a host cell provided by this invention containing the encoding nucleotide sequence of said fusion protein comprising regions A and B, and the encoding nucleotide sequence of IBDV pVP2, either in a single gene construct or in two different gene constructs, and simultaneously expressing said IBDV pVP2 proteins and fusion protein comprising regions A and B, and if desired, recovering said CVLPs of the invention. In a particular embodiment, said host cell provided by this invention is a cell that is transformed, transfected or infected with a suitable expression system, such as an expression system comprising a gene construct, wherein said gene construct comprises the nucleotide sequence encoding for said fusion protein comprising regions A and B, and the nucleotide sequence encoding for IBDV pVP2, or else alternatively with an expression system comprising a first gene construct comprising the nucleotide sequence encoding for said fusion protein comprising regions A and B, and a second gene construct comprising the nucleotide sequence encoding for IBDV pVP2.

Said process therefore comprises the gene coexpression of said IBDV pVP2 proteins and fusion proteins comprising regions A and B as two independent genes. After the simultaneous expression of said proteins (IBDV pVP2 and fusion proteins comprising regions A and B) in said cells, the expressed proteins are assembled and form the CVLPs of the invention, which can be isolated or withdrawn from the medium and purified if desired. The isolation and purification of said CVLPs of the invention can be carried out by conventional methods, for example, by means of fractioning on sucrose gradients.

In a particular embodiment, the simultaneous gene coexpression of IBDV pVP2 proteins and fusion proteins comprising regions A and B is carried out by means of the use of an rBV allowing the simultaneous expression of said proteins from two independent chimeric genes in insect cells. In this case, the process for the production of CVLPs of the invention provided by this invention comprises, first the obtainment of a gene expression system constituted by an rBV containing a gene construct simultaneously encoding for the IBDV pVP2 proteins and for said fusion proteins comprising regions A and B, such as the rBV called FBD/pVP2-his-VP3 (Example 1.2), or alternatively the obtainment of an rBV containing a gene construct encoding for the IBDV pVP2 protein and the obtainment of another rBV containing a gene construct encoding for said fusion protein comprising regions A and B, such as the rBVs called FB/pVP2 and FB/his-VP3 (Example 1.1), or rBVs called FB/pPV2 and FB/his-CD8-VP3 (Example 3), respectively, followed by the infection of insect cells with said expression system based on said recombinant baculovirus(es), expression of the recombinant proteins and if desired, isolation of the CVLPs of the invention formed by assembly of said IBDV pVP2 proteins and fusion proteins comprising regions A and B, and optionally subsequent purification of said CVLPs of the invention.

The construction of a recombinant baculovirus allowing the independent expression of the IBDV pVP2 proteins and the fusion proteins comprising regions A and B can be carried out by any person skilled in the art based on that described herein and on the state of the art concerning this technology (Cold Spring Harbor, N.Y.; Leusch MS, Lee SC, Olins PO. 1995. A novel host-vector system for direct selection of recombinant baculoviruses (bacmids) in Escherichia coli. Gene 160: 191-4; Luckow VA, Lee SC, Barry GF, Olins PO. 1993. Efficient generation of infectious recombinant baculoviruses by site-specific transposon-mediated insertion of foreign genes into a baculovirus genome propagated in Escherichia coli. J Virol 67: 4566-79).

In another particular embodiment, the gene coexpression of the IBDV pVP2 proteins and of the previously defined fusion proteins comprising regions A and B is carried out by means of the use of a vector allowing the expression of said proteins in yeast cells. In this case, the process for the production of CVLPs of the invention provided by this invention comprises, first, the obtainment of a gene expression system constituted by a plasmid containing a gene construct simultaneously encoding for the IBDV pVP2 proteins and for said fusion proteins comprising regions A and B, followed by the transformation of yeasts with said expression system, expression of the recombinant proteins, and if desired, isolation of the CVLPs of the invention formed by assembly of said IBDV pVP2 proteins and fusion proteins comprising regions A and B, and optionally subsequent purification of said CVLPs of the invention. In a specific embodiment, the suitable expression system for transforming yeasts is based on a pESC Yeast (Stratagene) expression system such as, for example, the plasmid pESCURA/pVP2/VP3-GFP (Example 2) containing a gene construct encoding for the IBDV pVP2 and VP3-GFP proteins.

The obtainment of yeasts transformed with a gene construct or with a suitable expression system or vector allowing the simultaneous expression of the IBDV pVP2 proteins and the fusion proteins comprising regions A and B can be carried out by any person skilled in the art based on that described herein and on the state of the art concerning this technology (pESC epitope tagging vectors Instructions manual. Stratagene www.stratagene.com; Sambrook, J., Fritsch, E.F., and Maniatis, T. (1989). Molecular cloning: a laboratory manual, 2nd ed. Cold Spring Harbor Laboratory).

In another aspect, the invention is related to the use of the gene expression system provided by this invention for producing and obtaining the CVLPs of the invention.

The CVLPs of the invention can be used as vectors or vehicles of products of interest, such as molecules with biological activity, for example, drugs, polypeptides, proteins, nucleic acids, etc., whereby they can be used for therapeutic or diagnostic or research purposes. In a particular embodiment, said molecules of biological interest include polypeptides of interest, such as antigens or immune response inducers in animals or humans to whom they are supplied, or nucleic acid sequences, useful in gene therapy, intended for being introduced inside the suitable cells.

Therefore, in another aspect, the invention is related to the use of the CVLPs of the invention in the manufacture of medicaments, for example vaccines, gene therapy vectors (delivery systems), etc. In a particular embodiment, said medicament is a vaccine intended for conferring protection against human or animal diseases caused by viruses, bacteria, parasites, or any other type of microorganism, or against tumor diseases. In another particular embodiment, said medicament is a gene therapy vector.

In another aspect, the invention provides a vaccine comprising a therapeutically effective amount of CVLPs of the invention, optionally together with one or more pharmaceutically acceptable adjuvants and/or vehicles. Said vaccine is useful for protecting animals and humans against diseases caused by microorganisms (viruses, bacteria, parasites, etc.), or against tumor diseases. In a particular embodiment, said vaccine is especially useful for simultaneously protecting animals or humans against the infection caused by two or more infectious disease-causing agents. By way of illustration, the vaccine provided by this invention can be used to protect birds, for example chickens, turkeys, geese, pheasants, quails, ostriches, etc., against the infectious bursal disease virus (IBDV) and against one or more infectious agents responsible for avian diseases (avian pathogens).

In the sense used in this description, the expression "therapeutically effective amount" refers to the amount of CVLPs of the invention calculated for producing the desired effect and will generally be determined, among others, by the characteristics of the CVLPs and the immunization effect to be achieved.

The pharmaceutically acceptable adjuvants and vehicles which can be used in said vaccines are those adjuvants and vehicles known by the persons skilled in the art and normally used in the manufacture of vaccines.

In a particular embodiment, said vaccine is prepared in form of an aqueous solution or suspension in a pharmaceutically acceptable diluent, such as saline solution, phosphate-buffered saline solution (PBS), or any other pharmaceutically acceptable diluent.

The vaccine provided by this invention can be administered by any suitable administration route which results in a protective immune response against the heterologous sequence or epitope used, to which end said vaccine will be formulated in the dosage form suited to the chosen administration route. In a particular embodiment, the administration of the vaccine provided by this invention is carried out parenterally, for example, intraperitoneally, subcutaneously, etc.

The following Examples illustrate the invention and should not be considered limiting of the scope thereof.

### EXAMPLE 1

### Obtaining IBDV CVLPs in insect cells

### 1.1 Obtaining IBDV CVLPs, VP2-his-VP3, by means of two independent rBVs in insect cells

The results of a series of experiments designed to analyze the possibility of obtaining IBDV CVLPs from the coexpression of the IBDV pVP2 and VP3 proteins and a heterologous polypeptide from two independent chimeric genes are described in this example. To that end, two recombinant baculoviruses (rBVs) described above, FB/his-VP3 (Kochan, G., González, D. & Rodríguez, J. F. (2003). Characterization of the RNA binding activity of VP3, a major structural protein of IBDV. Archives of Virology 148, 723-744) and FB/VPX, herein cited as FB/pVP2, (Martinez-Torrecuadrada, J. L., Castón, J. R., Castro, M., Carrascosa, J. L., Rodriguez, J. F. & Casal, J. I. (2000). Different architectures in the assembly of infectious bursal disease virus capsid proteins expressed in insect cells. Virology 278, 322-331) have been used. These rBVs were generated by means of the cloning into suitable vectors of the complementary DNA (cDNA) encoders of the IBDV pVP2 and pVP3 proteins. Said cDNAs were obtained by RT-PCR from the A segment of the serotype I IBDV Soroa strain genome a (NCBI access number AAD30136). The rBV FB/his-VP3 expresses a chimeric VP3 protein which at its N-termi.nal end contains a tandem of six histidines fused to the VP3 sequence (Met754-Glul012 of the polyprotein) called his-VP3. rBV FB/pVP2 expresses the encoding region of the pVP2 protein (Met1-Ala512).

The analysis of the expression of these pVP2 and his-pVP3 proteins, whether independently or together, was carried out in cell cultures. To carry out these experiments, single layer cell cultures from the insect *Trichloplusia ni* (H5, Invitrogen) were used, which were grown on cover glasses. Said cultures were independently infected with FB/pVP2, FB/his-VP3, or coinfected with both rBVs. The multiplicity of infection was 5 pfu/cell. The cells were fixed at 48 hours post-infection (h.p.i.), and incubated with rabbit anti-VP2 polyclonal serum and with rat anti-VP3 polyclonal serum (Fernández-Arias, A., Risco, C., Martinez, S., Albar, J. P. & Rodriguez, J. F. (1998). Expression of ORF A1 of infectious bursal disease virus results in the formation of virus-like particles. Journal of General Virology 79, 1047-1054). After successive washings, the cover glasses were incubated with goat anti-rat serum conjugated with Alexa 594 and goat anti-rabbit serum conjugated with Alexa 488 (Jackson Immunoresearch Laboratories, Inc.). The cellular cores were stained with the specific To-Pro-3 marker (Molecular Probes, Inc.). The samples were finally viewed by epifluorescence with a Zeiss Axiovert 200 microscope equipped with the Bio Rad Radiance 2100 confocal system. The images obtained were stored using the Laser Sharp Package (Bio Rad) software equipment. As is shown in Figure 2a, in the cultures infected with FB/pVP2, the anti-VP2 serum showed a fine granular signal mixed with tubular structures, both distributed throughout the cytoplasm. The anti-VP3 signal, detected in the cells infected with rBV FB/his-VP3, was characterized by the presence of spherical-shaped, and apparently hollow, accumulations around the core. In the cultures coinfected with both rBVs, a notable modification in the distribution pattern of both proteins was detected. In these cells, the specific signals of pVP2 and VP3 were collocated in spherical and dense accumulations, suggesting that their coexpression allowed the formation of pVP2/his-VP3 complexes (Figure 2c to 2e).

For the purpose of characterizing these structures in greater detail, similar extracts corresponding to cells infected with FB/pVP2+FB/hisVP3 were analyzed by transmission electron microscopy (TEM). As a control, and in parallel, H5 cell cultures infected with the wild strain of the FBD (FastBacDual, Invitrogen) virus were analyzed by the same technique. After the infection, the cells were harvested after 48 hours, and processed as has been previously described (Fernández-Arias A, Risco C, Martinez S, Albar JP & Rodriguez JF. (1998). Expression of ORF A1 of infectious bursal disease virus results in the formation of virus-like particles. Journal of General Virology 79:1047-1054) for their analysis in ultrathin sections by TRANSMISSION ELECTRON MICROSCOPY. As is shown in Figure 2, the cytoplasm of the coinfected cells contains aggregates formed by a mixture of tubules and structures similar to capsids (Figure 2g, 2h and 2i). These aggregates were not observed in any case in the samples corresponding to cells infected with wild FBD virus (Figure 2f). The appearance and size of the tubules, as well as of the structures similar to capsids, was similar to those previously described in cell cultures infected with VT7/Poly, a recombinant of the vaccinia virus expressing the gene of the IBDV polyprotein (Fernández-Arias A, Risco C, Martinez S, Albar JP & Rodriguez JF. (1998). Expression of ORF A1 of infectious bursal disease virus results in the formation of virus-like particles. Journal of General Virology 79:1047-1054).

To unmistakably establish that the coexpression of pVP2 and his-VP3 enabled the assembly and, therefore, the obtainment of CVLPs, the decision was made to purify the formed particles. To that end, H5 cell cultures were infected with FB/pVP2+FB/his-VP3. At 60 h.p.i., the cells were homogenized and the extracts were separated on sucrose gradients as previously described (Lombardo E, Maraver A, Castón JR, Rivera J, Fernández-Arias A, Serrano A, Carrascosa JL & Rodriguez JF. (1999). VP1, the putative RNA-dependent RNA polymerase of infectious bursal disease virus, forms complexes with the capsid protein VP3, leading to efficient encapsidation into virus-like particles. Journal of Virology 73:6973-6983). After their centrifugation, the gradients were fractioned, and the different fractions were analyzed by TEM as previously described (Lombardo E, Maraver A, Castón JR, Rivera J, Fernández-Arias A, Serrano A, Carrascosa JL & Rodriguez JF. (1999). VP1, the putative RNA-dependent RNA polymerase of infectious bursal disease virus, forms complexes with the capsid protein VP3, leading to efficient encapsidation into virus-like particles. Journal of Virology 73:6973-6983). As a control, and subject to the same process, gradients corresponding to cell extracts infected with rBV FB/VPX or with rBV FBD/Poly-VP1, were fractioned. The recombinant virus FBD/Poly-VP1 simultaneously expresses the VP1 polypeptide and polyprotein. As was predictable, the infection with FBD/Poly-VP1 had a result of an efficient production of VLPs (Maraver A, Oña A, Abaitua F, González D, Clemente R, Diaz-Ruiz A, Castón JR, Pazos F & Rodríguez JF. (2003). The oligomerization domain of VP3, the scaffolding protein of infectious bursal disease virus, plays a critical role for capsid formation. Journal of Virology 77:6438-49). On the other hand, the fractions corresponding to the cells infected with FB/VPX only contain tubules of a twisted appearance. The gradients corresponding to cells coinfected with the rBVs FB/pVP2+FB/his-VP3 contain rigid type I tubules in the fractions near the bottom of the gradient, and CVLPs in the central and top fractions (Figure 3b). The CVLPs isolated from the cells coinfected with rBV FB/pVP2+FB/his-VP3 had a diameter of 65-70 nm, as well as a typical polygonal contour, absolutely indistinguishable from the purified VLPs of cultures infected with FBD/Poly-VP1 (Maraver, A., Oña, A., Abaitua, F., González, D., Clemente, R., Diaz-Ruiz, A., Caston, J. R., Pazos, F. & Rodriguez, J. F. (2003). The oligomerization domain of VP3, the scaffolding protein of infectious bursal disease virus, plays a critical role for capsid formation. Journal of Virology 77:6438-49) or of the cultures infected with VT7/Poly (Fernández-Arias, A., Risco, C., Martinez, S., Albar, J. P. & Rodriguez, J. F. (1998). Expression of ORF A1 of infectious bursal disease virus results in the formation of virus-like particles. Journal of General Virology 79, 1047-1054).

For the purpose of achieving a biochemical characterization of the obtained material, Western blot experiments were carried out in which the different fractions were compared with specific sera against the VP1, pVP2, VP3 and VP4 proteins (Fernández-Arias *et al.* 1998, cited *supra;* Lombardo *et al.,* 2000). Cell extracts infected with IBDV were used as a control. The obtained results are shown in Figure 3d. As was expected, the bands corresponding to the VP1 and VP4 polypeptides were only detected in samples corresponding to cells infected with FBD/Poly-VP1. The patterns corresponding to pVP2/VP3 in simples corresponding to cells infected with FBD/Poly-VP1 or coinfected with FB/VPX+ FB/his-VP3 were similar, two bands corresponding to pVP2 and VP3, respectively, being detected.

### 1.2 Obtaining IBDV CVLPs, pVP2-his-VP3, by means of a single rBV in insect cells

Furthermore, the construction of the plasmid pFBD/pVP2-his-VP3 was carried out. The first step of the construction was carried out by means of the cloning of the encoding region of the pVP2 protein into the pFBDual vector (Invitrogen). The DNA fragment corresponding to pVP2 was obtained by means of PCR with the oligonucleotides identified as Oligo I (SEQ ID NO: 1) and Oligo II (SEQ ID NO: 2) using the plasmid pVOTE.2/Poly as a mold (Fernández-Arias, A., Risco, C., Martinez, S., Albar, J. P. & Rodriguez, J. F. (1998). Expression of ORF A1 of infectious bursal disease virus results in the formation of virus-like particles. Journal of General Virology 79, 1047-1054). The fragment was purified, subjected to digestion with the BgIII and HindIII enzymes and cloned into the pFBDual vector (Invitrogen) previously digested with the BamHI and Hindi enzymes. The resulting plasmid was called pFBD/pVP2. Then, a DNA fragment containing the open reading frame corresponding to the VP3 protein was obtained by means of digestion of the plasmid pFB/his-VP3 (Kochan et al., 2003, cited *supra*) with the RsrII enzyme, treatment with Klenow, and subsequent restriction with KpnI. This DNA fragment was purified and cloned into the plasmid pFBD/pVP2 previously digested with the SmaI and KpnI enzymes. The resulting plasmid was called pFBD/pVP2-his-VP3 (SEQ ID NO: 3) and contains the encoding nucleotide sequence of the pVP2 proteins and of the his-pVP3 fusion protein containing a heterologous his 6 sequence (the latter is encoded by the complementary chain to the nucleotides 6734-7585 of SEQ ID NO: 3). The amino acid sequence of the pVP2 protein and of the his-VP3 fusion protein (pVP2-his-VP3) encoded by the nucleotide sequence contained in said plasmid pFBD/pVP2-his-VP3 is shown in SEQ ID NO: 4.

The plasmid pFBD/pVP2-his-VP3 allows obtaining an rBV, called FBD/pVP2-his-VP3, expressing both proteins simultaneously during its replication cycle [http://invitrogen.com/content/sfs/manuals/bevtest.pdf].

The results obtained with FBD/pVP2-his-VP3 in insect cells are identical to those obtained by means of the coinfection with rBVs FB/pVP2 and FD/his-VP3, IBDV CVLPs with the heterologous six histidine (6 his) polypeptide being obtained.

### EXAMPLE 2

### Obtaining IBDV CVLPs, pVP2-VP3-GFP, in yeasts

For the purpose of studying the possibility of obtaining IBDV CVLPs in yeast cultures (*Saccharomyces cerevisiae*) the vector pESCURA/pVP2-VP3-GFP was generated with the heterologous GFP gene bound to the VP3 N-terminal end. The first step in the construction of the vector was carried out by means of the cloning of the encoding region of the pVP2 protein into the vector pESCURAinv. The plasmid pESCURAinv was generated by means of digestion of the vector pRS426 (Stratagene) with the PvuII enzyme and religation of the digestion mixture. The resulting vector, pESCURAinv, contains the multiple cloning region in reversed position with regard to that of parent vector pRS426. The DNA fragment corresponding to the pVP2 protein was obtained by means of PCR with the oligonucleotides called Oligo III (SEQ ID NO: 5) and Oligo IV (SEQ ID NO: 6) using the plasmid pVOTE.2/Poly as a mold (Fernández-Arias, A., Risco, C., Martínez, S., Albar, J. P. & Rodríguez, J. F. (1998). Expression of ORF A1 of infectious bursal disease virus results in the formation of virus-like particles. Journal of General Virology 79, 1047-1054). The fragment was purified subjected to digestion with the BgIII and HindIII enzymes and cloned into the vector pESCURA.inv, previously digested with the BamHI and HindIII enzymes. The resulting plasmid was called pESCUR.A/pVP2.

The plasmid pFB/VP3-GFP was constructed in two stages. The first one consisted of the cloning of a DNA fragment, generated by means of PCR, containing the ORF of the VP3 protein lacking the termination codon. This PCR was carried out using the oligonucleotides called Oligo V (SEQ ID NO: 9) and Oligo VI (SEQ ID NO: 10) and using the plasmid pVOTE.2/Poly as a mold (Fernández-Arias, A., Risco, C., Martinez, S., Albar, J. P. & Rodriguez, J. F. (1998). Expression of ORF A1 of infectious bursal disease virus results in the formation of virus-like particles. Journal of General Virology 79, 1047-1054). The resulting DNA was digested with the EcoRI and BamHI enzymes and cloned into the vector pEGFP-N3 (Clontech), also digested with the same enzymes. The resulting plasmid was called pVP3-GFP. Then, the plasmid pEGFP-GFP was digested with the EcoRI and NotI enzymes and cloned into the vector pFastBacl (Invitrogen). The resulting plasmid was called pFB/VP3-GFP.

Next, a DNA fragment that contained the open reading frame corresponding to the VP3 protein fused to the encoding region of the EGFP protein was obtained by means of digestion of the plasmid pFB/VP3-GFP with the EcoRI and NotI enzymes. This DNA fragment was purified and cloned into the plasmid pESCUR.A/pVP2 previously digested with the EcoRI and NotI enzymes. The resulting plasmid was called pESCURA/pVP2-VP3-GFP (SEQ ID NO: 7) and contains the ORFs of the pVP2 and VP3-GFP proteins under the transcriptional control of two independent promoters, GAL 1 and GAL 10, both inducible by galactose (the pVP2 protein is encoded by the chain of nucleotides complementary to the nucleotides 5862-7343 of SEQ ID NO: 7). The amino acid sequence of the pVP2 protein and of the VP3-GFP fusion protein (pVP2-VP3-GFP) encoded by the nucleotide sequence contained in said plasmid pESCURA/pVP2-VP3-GFP is shown in SEQ ID NO: 8.

pESCURA/pVP2-VP3-GFP was subsequently used to transform a culture of *S. cerevisiae* yeast haploid strain 499 according to a previously described protocol (Gietz, R.D. and R.A. Woods. (2002), Transformation of yeast by the Liac/SS carrier DNA/PEG method. Methods in Enzymology 350:87-96). The yeasts transformed with the plasmid were selected by means of growth on SC medium plates (CSM + YNB, 2% glucose and bacto agar) supplemented with the amino acids tryptophan, leucine and histidine and lacking uracyl (-Ura). After an incubation of 48 hours at 30°C, a colony was chosen which was used to carry out the following protein expression and CVLP formation analyses.

The pVP2 and VP3 protein expression and,CVLP formation analyses were carried out following a protocol previously described for the characterization of IBDV VLPs in other expression systems (Fernández-Arias, A., Risco, C., Martinez, S., Albar, J. P. & Rodriguez, J. F. (1998). Expression of ORF A1 of infectious bursal disease virus results in the formation of virus-like particles. Journal of General Virology 79,1047-1054; Lombardo, E., Maraver, A., Castón, J. R., Rivera, J., Fernández-Arias, A., Serrano, A., Carrascosa, J. L. & Rodriguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-698). The colony selected was cultured in liquid CSM (-Ura) + YNB medium supplemented with 2% raffinose. The culture was incubated at 30°C for 24 hours. This culture was used to inoculate, at an optical density (O.D.) of 0.2, a flask of 200 ml of CSM (-Ura) + YNB medium supplemented with 2% inducer galactose. The culture was maintained at 30°C for 18 hours (until an O.D. between 1.0 and 2.0). The yeasts were centrifuged at 3,000 radiant power measurement, 5 minutes at 4°C, were washed once with distilled water, and the pellet was resuspended in lysis buffer (TEN: Tris 10 mM, pH 8.0; NaCl 150 mM; EDTA 1 mM) + 2X protease inhibitors (Compl Roche). A volume of glass beads having a size of about 425-600 microns (Sigma) were added for the lysis. This mixture was subjected to vigorous vortex stirring for 30 seconds 4 times, with 30-second intervals, and at 4°C. After this, the soluble fraction was recovered by centrifuging the lysis mixture at 13,000 rpm for 15 minutes at 4°C. This sample was subjected to fractioning on a sucrose gradient according to a previously described protocol (Lombardo, E., Maraver, A., Castón, J. R., Rivera, J., Fernández-Arias, A., Serrano, A., Carrascosa, J. L. & Rodriguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-6983). The samples obtained after fractioning as well as a sample of the starting material were analyzed by means of sodium dodecylsulfate polyacrylamide gel electrophoresis (SDS-PAGE) [Current Protocols in Molecular Biology] and immunodetection by Western blot (Figure 4A) using anti-pVP2 and anti-VP3 sera [Current Protocols in Molecular Biology]. As is shown in Figure 4A, the Western blot showed the presence of bands, with the predicted molecular mass corresponding to the pVP2 (48 kDa) and VP3-GFP (61 kDa) proteins, as well as other immunoreactive bands of a smaller size probably produced by proteolytic degradation both in the initial sample and in the different fractions of the gradient. These results reliably showed the correct expression of both polypeptides in the *S. cerevisiae* culture transformed with the plasmid pESCURA/pVP2-VP3. Then, the different fractions of the gradient were analyzed by means of TEM as has been previously described (Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73, 6973-6983). As is shown in Figure 4B, the TEM analysis of the fractions of the gradient showed the existence of IBDV CVLPs in the top fractions of the gradient. These CVLPs have a diameter of 65-70 nm and a polygonal contour that is indistinguishable from the IBDV CVLPs obtained in other expression systems (Figure 4C).

### EXAMPLE 3

### Obtaining and characterizing the immunogenicity of IBDV CVLPs

As part of the development of new vaccination strategies, the possibility of using the strategy of producing chimeric IBDV VLPs (CVLPs) which contained heterologous amino acid sequences corresponding to other proteins or peptides involved in the induction of an immune response was analyzed. As a study model, the possibility of obtaining CVLPs which contained, as a heterologous polypeptide comprising a polypeptide of interest, the amino acid sequence corresponding to the CD8 epitope (E-CD8) of the malaria CS protein (*Plasmodium yoelii*), was approached. (Quantification of antigen specific CD8+ T cells using an ELISPOT assay. J Immunol Methods 181: 45-54; Zavala, F., Rodrigues, M., Rodriguez, D., Rodriguez, J. R., Nussenzweig, R. S. and Esteban, M. (2001). A striking property of recombinant poxviruses: efficient inducers of in vivo expansion of primed CD8(+) T cells. Virology 280: 155-159). This epitope is responsible for the CD8-specific cellular immune response induction against this pathogen (Oliveira-Ferreira J, Miyahira Y, Layton GT, Savage N, Esteban M, Rodriguez D, Rodriguez JR, Nussenzweig RS, Zavala F, Myahira Y. (2000). Immunogenicity of Ty-VLP bearing a CD8(+) T cell epitope of the CS protein of P. yoelii: enhanced memory response by boosting with recombinant vaccinia virus. Vaccine 18: 1863-1869). This response can be quantified by means of the ELISPOT technique (Miyahira Y, Murata K, Rodriguez D, Rodriguez JR, Esteban M, Rodrigues MM, Zavala F. (1995) Quantification of antigen specific CD8+ T cells using an ELISPOT assay. J Immunol Methods 181: 45-54) in splenocyte cultures from BALB/c mice.

For this purpose, the construction of the plasmid pFB/his-CD8-VP3 (SEQ ID NO: 13) was carried out following the cloning strategy described later. This vector was constructed by means of insertion of a 36 bearing portion DNA fragment, generated by means of hybridization of the synthetic oligonucleotides identified as CD8 A (SEQ ID NO: 11) and CD8 B (SEQ ID NO: 12), containing the encoding sequence of the CD8 epitope (SYVPSAEQI, see residues 29 to 37 of the SEQ ID NO: 13 and 14) of the malaria CS protein in the ORF encoding the his-VP3 protein integrated in the pFB/his-VP3 vector. The cloning was carried out by means of ligation of the DNA fragment generated by means of hybridization of the synthetic oligonucleotides CD8 A and B (SEQ ID NO: 11 and SEQ ID NO: 12) to the plasmid pFB/his-VP3 digested with the EheI restriction enzyme. This plasmid pFB/his-CD8-VP3 (SEQ ID NO: 13) contains and ORF encoding a fusion protein called his-CD8-VP3 containing the CD8 epitope inserted in the end corresponding to the N-terminal sequence of the his-VP3 protein ORF. The amino acid sequence of the his-CD8-VP3 fusion protein encoded by the nucleotide sequence contained in said plasmid pFB/his-CD8-VP3 is shown in SEQ ID NO: 14.

The plasmid pFB/his-CD8-VP3 was purified and used to generate the corresponding recombinant baculovirus (rBV), called FB/his-CD8-VP3, following the Bac-to-Bac technology according to the protocols described by the manufacturer (Invitrogen BV, Groningen, The Netherlands).

### 3.1 Producing CVLPs

H5 cell cultures were simultaneously infected with the recombinant baculoviruses FB/His-CD8-VP3 and FB/pVP2. The FB/ pVP2 rBV (see Example 1.1) expresses the region corresponding to the pVP2 protein (Met1-Ala 512) of the IBDV polyprotein. The cells were harvested at 48 hours post-infection (pi), and the corresponding extracts were subjected to the IBDV VLPs purification protocol by means of fractioning on linear sucrose gradients (Lombardo, E., Maraver, A., Castón, J. R., Rivera, J., Fernández-Arias, A., Serrano, A., Carrascosa, J. L. & Rodríguez, J. F. (1999). VP1, the putative RNA-dependent RNA polymerase of infectious bursal disease virus, forms complexes with the capsid protein VP3, leading to efficient encapsidation into virus-like particles. Journal of Virology 73: 6973-6983). Each one of the obtained fractions was viewed by means of transmission electron microscopy (TEM) and analyzed by means of SDS-PAGE and immunoblot using VP3 specific antibody. As is observed in Figure 5A, fraction 4 of the gradient contained abundant assemblies with an identical structure (polygonal perimeter and a diameter of 65-70 nm) as the IBDV VLPs obtained by means of expression of the viral polyprotein. The biochemical characterization, by means of SDS-PAGE and Western blot (Figure 5B), showed that these CVLPs contain a protein, immunoreactive against the anti-VP3 serum, the molecular mass (33.5 kDa) of which is identical to the aforementioned one for the his-CD8-VP3 fusion protein (33.591 kDa). These results allow concluding that the coexpression of the pVP2 and his-CD8-VP3 genes in insect cells gives rise to the formation of chimeric VLPs (CVLPs) containing the his-CD8-VP3 fusion protein. These CVLPs are called CD8-CVLPs.

### 3.2 Immunogenicity analysis of CD8-CVLPs

For the purpose of determining the immunogenic capacity of the CD8-CVLPs two identical assays were carried out using two batches of CD8-CVLPs independently produced and purified. Four groups (I, II, III and IV) of three female eight-week old BalbC rats were used. The groups were formed randomly. The immunization strategy was similar to the one used previously in the characterization of other immunogens. This strategy is based on the use of a priming dose with the antigen under study, followed by a second booster dose, which amplifies the primary response, with the recombinant vaccinia virus VVpJRCS, which expresses the malaria CS protein. The induced immune response was determined by means of the detection of the antigen specific CD8⁺ T cells according to their ability to produce IFN-γ, by means of an ELISPOT assay (Miyahira Y, Murata K, Rodriguez D, Rodriguez JR, Esteban M, Rodrigues MM, Zavala F. (1995). Quantification of antigen specific CD8+ T cells using an ELISPOT assay. J Immunol Methods 181: 45-54). In summary, 96-well plates with nitrocellulose (Millipore) bottoms were coated with 75 µl/well of a solution containing 6 µg/ml of the rat anti-murine IFN-γ monoclonal antibody (R4-6A2, Pharmingen, San Diego, CA) resuspended in PBS. The plates were incubated overnight at room temperature. The wells were subsequently washed three times with RPMI medium, and were finally incubated with RPMI medium supplemented with 10% fetal calf serum (FCS) for one hour at 37°C 5% CO₂ atmosphere. On the other hand, the spleens of the immunized rats, maintained in RPMI medium supplemented with 10% FCS, were arranged on a sterile grid on a 60 movable member plate and were homogenized, the extract breaking up by means of its passing through needles of different gauges (21G->25G). The cells thus broken up were centrifuged for 5 minutes at 1,500 rpm at 4°C, and were washed twice with RPMI + 10% FCS medium. In order to lysate the erythrocytes of the samples, sterile 0.1 M NH₄Cl (2 ml/spleen) was added and it was maintained at 4°C for 3-5 minutes, RPMI + 10% FCS was added and it was centrifuged. Then, they were twice and it was finally resuspended in 1-2 ml RPMI + 10% FCS. The splenocyte viability count was carried out by means of trypan blue staining (4% in water, Sigma).

The professional antigen-presenting cells (APCs) used in this assay were P815. These cells were adjusted to a concentration of 10⁶ cells/ml and were incubated with the synthetic peptide SYVPSAEQI (corresponding to the CD8 region of the malaria CS protein) 10⁻⁶ M. After treatment with the peptide, the cells were washed and treated with mitomycin C (30 µg/ml) (Sigma) for 15 minutes at 37°C and in CO₂ atmosphere. After subsequent washings, the antigen-presenting cells, to which 30 U/ml of murine interleukin 2 (IL-2) were added, were added at a concentration of 10⁵ cells/well. 100 µl/well of 10⁶ splenocytes/ml and 1/4 and 1/16 dilutions were also added. The plates were incubated for 18 ours at 37°C in CO₂ atmosphere, they were washed 5 times with PBST and incubated with 2 µg/ml of the biotinylated rat anti-IFN-γ XMG1.2 monoclonal antibody (Pharmingen) diluted in PBST for 2 hours at room temperature. Then the plates were washed five times with PBST and a dilution of 1/800 avidin-peroxidase was added (0.5 mg/ml) (Sigma). After 1 hour of incubation at room temperature, it was washed 3 times with PBST and 2 times with PBS, finally adding the developer mixture with 1 µg/ml of the DAB substrate (Sigma), resuspended in Tris-HCl, pH 7.5, 50 mM, containing 0.015% H₂O₂. The reaction was stopped by washing the plate with abundant water, and once dried, the spots were counted with the aid of a Leica MZ122 APO stereomicroscope and the QWIN Imaging System software (Leica, Cambridge, United kingdom).

Immunizations were carried out according to the immunization program described in the following table:

| Group | 1^{st} Immunization. Day O | 2^{nd} Immunization. Day 14 |
|---|---|---|
| I | Not immunized | VVpJRPyCS |
| II | VVpJRPyCS | VVpJRPyCS |
| III | IBDV VLPs | VVpJRPyCS |
| IV | CD8-CVLPs | VVpJRPyCS |

Immunizations with VVpJRCS were carried out intraperitoneally using 10⁷ plaque forming units (pfu) per anima. Immunizations with VLPs, both non-chimeric IBDV VLPs and CDS-CVLPs, were carried out intraperitoneally with a dose of 50 µg of antigen per animal. In all cases, the antigen preparations were diluted in phosphate-buffered saline (PBS).

28 days after the first immunization, the animals were sacrificed and there spleens were used to carry out the ELISPOT assays. These assays were carried out following the protocol described above. Virtually identical results were obtained in both assays. Figure 6 shows the results corresponding to the first assay. The obtained results demonstrate that when CD8-CVLPs are used as a priming dose followed by a booster dose with the WpJRCS virus (group IV), a strong stimulation of the specific cellular immune response against the malaria CD8 epitope occurs. This stimulation is much greater (about 20 times greater) than that obtained after the immunization with one (group I) or two (group II)doses of VVpJR.CS. The fact that a significant stimulation of the response against E-CD8 dose not occur in animals immunized with non-chimeric IBDV VLPs (group III), with regard to group I, which received a single dose of WpJRCS, demonstrates that the response obtained in group IV is specifically induced by the E-CD8 present in the his-CD8-VP3 fusion protein forming an integral part of the CD8-CVLPs.

## Claims

1. A chimeric empty capsid of the infectious bursal disease virus (IBDV), **characterized in that** it is constituted by assembly of (i) IBDV pVP2 proteins and (ii) fusion proteins comprising a region A constituted by the IBDV VP3 protein bound to a region B constituted by a heterologous polypeptide comprising a polypeptide of interest, wherein said region B is bound to the amino-terminal region of IBDV VP3.

2. Capsid according to claim 1, wherein said polypeptide of interest is a polypeptide useful in vaccination, therapy or diagnosis.

3. Capsid according to claim 1, wherein said region B comprises a single polypeptide of interest.

4. Capsid according to claim 1, wherein said region B comprises two or more polypeptides of interest.

5. Capsid according to claim 1, wherein said fusion protein comprises a region A bound to a single region B.

6. Capsid according to claim 1, wherein said fusion protein comprises a region A bound to two regions B, equal or different, one of them bound to the amino-terminal region of VP3 present in region A, and the other one to the carboxy-terminal region of VP3 present in region A.

7. Capsid according to claim 6, wherein said regions B contain more than one polypeptides of interest equal to or different from one another.

8. Capsid according to claim 1, wherein said fusion protein further comprises, a linker polypeptide located between said regions A and B.

9. A nucleic acid comprising a nucleotide sequence encoding for the chimeric empty capsids of claims 1 to 8, which comprises the nucleotide sequence encoding for the fusion protein defined in anyone of claims 1 to 8, further comprising (iii) a nucleotide sequence comprising the open reading frame corresponding to the IBDV pVP2 protein.

10. A nucleic acid according to claim 9 wherein the nucleotide sequence encoding for the fusion protein, comprises (i) a nucleotide sequence comprising the open reading frame corresponding to the IBDV VP3 protein and (ii) a nucleotide sequence comprising the open reading frame of a heterologous polypeptide comprising a polypeptide of interest, wherein in the resulting fusion protein said heterologous polypeptide is bound to the amino-terminal region of IBDV VP3.

11. A gene construct comprising a nucleic acid according to claim 9 or 90.

12. An expression system selected from:
a) an expression system comprising a first gene construct comprising a nucleic acid which comprises (i) a nucleotide sequence comprising the open reading frame corresponding to the IBDV VP3 protein and (ii) a nucleotide sequence comprising the open reading frame of a heterologous polypeptide comprising a polypeptide of interest, wherein in the resulting fusion protein said heterologous polypeptide is bound to the amino-terminal region of IBDV VP3, operatively bound to transcription, and optionally translation, control elements, and a second gene construct, operatively bound to transcription, and optionally translation, control elements; said second gene construct comprising a nucleotide sequence comprising the open reading frame corresponding to the IBDV pVP2 protein; and
b) an expression system comprising a gene construct according to claim 11, operatively bound to transcription, and optionally translation, control elements.

13. Expression system according to claim 12, said expression system being selected from plasmids, bacmids, yeast artificial chromosomes (YACs), bacteria artificial chromosomes (BACs), bacteriophage PI-based artificial chromosomes (PACs), cosmids, or viruses, which optionally contain a heterologous replication origin.

14. A host cell containing a nucleic acid according to anyone of claims 9 or 10, or a gene construct according to claim 19, or an expression system according to anyone of claims 12 or 13.

15. A host cell, said cell having been transformed, transfected or infected with an expression system according to any of claims 12 or 13.

16. Host cell according to claims 14 or 15, said cell being selected from a mammal cell, an avian cell, an insect cell and a yeast.

17. A process for the production of chimeric empty capsids of the infectious bursal disease virus (IBDV) according to anyone of claims 1 to 8, comprising culturing a host cell according to anyone of claims 14 to 16, and, if desired, recovering said chimeric empty IBDV capsids.

18. Process according to claim 17, wherein said host cell is an insect cell, comprising the steps of:
a) preparing an expression system selected from (I) and (II), wherein:
- expression system (I) is constituted by a recombinant baculovirus containing a gene construct according to claim 19; and
- expression system (II) is constituted by a first recombinant baculovirus containing a gene construct encoding for the IBDV pVP2 protein, and a second recombinant baculovirus containing a gene construct comprising a nucleic acid which comprises (i) a nucleotide sequence comprising the open reading frame corresponding to the IBDV VP3 protein and (ii) a nucleotide sequence comprising the open reading frame of a heterologous polypeptide comprising a polypeptide of interest, wherein in the resulting fusion protein said heterologous polypeptide is bound to the amino-terminal region of IBDV VP3;
b) infecting insect cells with said expression system prepared in step a);
c) culturing the infected insect cells obtained in step b) under conditions allowing the expression of recombinant proteins and their assembly to form chimeric empty IBDV capsids; and
d) if desired, isolating and optionally purifying the chimeric empty IBDV capsids.

19. A process according to claim 17, wherein said host cell is a yeast, comprising the steps of;
a) preparing an expression system constituted by a plasmid containing a gene construct according to claim 11;
b) transforming yeast cells with said expression system prepared in step a);
c) culturing the transformed yeasts obtained in step b) under conditions allowing the expression of recombinant proteins and their assembly to form chimeric empty IBDV capsids; and
d) if desired, isolating and optionally purifying the chimeric empty IBDV capsids.

20. The use of a gene expression system according to anyone of claims 12 or 13 for producing chimeric empty IBDV capsids according to anyone of claims 1 to 8.

21. The use of chimeric empty capsids of the infectious bursal disease virus (IBDV) according to anyone of claims 1 to 8 in the manufacture of a medicament.

22. Use according to claim 21, wherein said medicament is a vaccine.

23. Use according to claim 21, wherein said medicament is a gene therapy vector.

24. A vaccine comprising a therapeutically effective amount of chimeric empty capsids of the infectious bursal disease virus (IBDV) according to anyone of claims 1 to 8, optionally together with one or more pharmaceutically acceptable adjuvants and/or vehicles.

25. A vaccine according to claim 24, useful to simultaneously protect animals or humans against infection caused by two or more disease-causing infectious agents.

26. A gene therapy vector comprising a chimeric empty capsid of the infectious bursal disease virus (IBDV) according to anyone of claims 1 to 8.

## Patentansprüche

1. Chimäres, leeres Kapsid des Virus der infektiösen Bursitis (IBDV), **dadurch gekennzeichnet, dass** es aus einer Anordnung von (i) IBDV-pVP2-Proteinen und (ii) Fusionsproteinen besteht, die einen Bereich A umfassen, der aus dem IBDV-VP3-Protein besteht, das mit einem Bereich B verbunden ist, der aus einem heterologen Polypeptid besteht, der ein interessierendes Polypetid umfasst, bei dem der genannte Bereich B mit dem aminoterminalen Bereich des IBDV-VP3 verbunden ist.

2. Kapsid nach Anspruch 1, bei dem das genannte interessierende Polypeptid ein bei der Impfung, Therapie oder Diagnose nützliches Polypeptid ist,

3. Kapsid nach Anspruch 1, bei dem der genannte Bereich B ein einzelnes interessierendes Polypeptid umfasst.

4. Kapsid nach Anspruch 1, bei dem der genannte Bereich B zwei oder mehr interessierende Polypeptide umfasst.

5. Kapsid nach Anspruch 1, bei dem das genannte Fusionsprotein einen Bereich A umfasst, der mit einem einzelnen Bereich B verbunden ist.

6. Kapsid nach Anspruch 1, bei dem das genannte Fusionsprotein einen Bereich A umfasst, der mit zwei gleichen oder unterschiedlichen Bereichen B verbunden ist, von denen einer mit dem aminoterminalen Bereich des im Bereich A vorhandenen VP3 verbunden ist, und der andere mit dem carboxy-terminalen Bereich des im Bereich A vorhandenen VP3.

7. Kapsid nach Anspruch 6, bei dem die genannten Bereiche B mehr als ein interessierendes Polypeptid enthalten, die untereinander gleich oder unterschiedlich sind.

8. Kapsid nach Anspruch 1, bei dem das genannte Fusionsprotein desweiteren ein zwischen den genannten Bereichen A und B gelegenes Linker-Polypeptid umfasst. ,

9. Nukleinsäure, welche eine Nukleotidsequenz umfasst, welche die chimären, leeren Kapside der Ansprüche 1 bis 8 codiert, welche die Nukleotidsequenz umfasst, die das in einem der Ansprüche 1 bis 8 definierte Fusionsprotein codiert, welche des weiteren (iii) eine Nukleotidsequenz umfasst, die den offenen Leserahmen entsprechend dem IBDV-pVP2-Protein umfasst.

10. Nukleinsäure nach Anspruch 9, bei der die das Fusionsprotein codierende Nukleotidsequenz (i) eine Nukleotidsequenz umfasst, welche den offenen Leserahmen entsprechend dem IBDV-VP3-Protein umfasst und (ii) eine Nukleotidsequenz, welche den offenen Leserahmen eines heterologen Poplypeptids umfasst, der ein interessierendes Polypeptid umfasst, bei der in dem resultierenden Fusionsprotein das genannte heterologe Polypeptid mit dem aminoterminalen Bereich des IBDV-VP3 verbunden ist.

11. Genkonstrukt, welches eine Nukleinsäure nach Anspruch 9 oder 10 umfasst.

12. Expressionssystem, ausgewählt aus:
a) einem Expressionssystem, das ein eine Nukleinsäure umfassendes erstes Genkonstrukt umfasst, welches (i) eine den offenen Leserahmen entsprechend dem IBDV-VP3-Protein umfassende Nukleotidsequenz umfasst, und (ii) eine den offenen Leserahmen eines heterologen Polypeptids umfassende Nukleotidsequenz, welcher ein interessierendes Polypeptid umfasst, bei dem in dem resultierenden Fusionsprotein das genannte heterologe Polypeptid mit dem aminoterminalen Bereich des IBDV-VP3 verbunden ist, der mit Transkriptionselementen und wahlweise mit Translations- und Kontrollelementen wirkverbunden ist und ein zweites Genkonstrukt, der mit Transkriptionselementen und wahlweise mit Translations- und Kontrollelementen wirkverbunden ist: wobei das genannte zweite Genkonstrukt eine den offenen Leserahmen entsprechend dem IBDV-pVP2-Protein umfassende Nukleotidsequenz umfasst; und
b) ein Expressionssystem, das ein Genkonstrukt nach Anspruch 11 umfasst, das mit Transkriptionselementen, und wahlweise mit Translations- und Kontrollelementen wirkverbunden ist.

13. Expressionssystem nach Anspruch 12, wobei das genannte Expressionssystem aus Plasmiden, Bacmiden, künstlichen Hefechromosomen (YAC's, Yeast Artificial Chromosomes), künstlichen Eakterienchromosomen (BAC's, Bacteria Artificial Chromosomes), künstlichen Chromosomen auf Grundlage des P1 aus Bakteriophage (PAC's, P1-based Artificial Chromosomes), Cosmiden, oder Viren ausgewählt wird, welche wahlweise einen heterologen Replikationsursprung enthalten.

14. Wirtszelle, welche eine Nukleinsäure nach einem der Ansprüche 9 oder 10, oder ein Genkonstrukt nach Anspruch 11, oder ein Expressionssystem nach einem der Ansprüche 12 oder 13 enthält.

15. Wirtszelle, wobei die genannte Zelle mit einem Expressionssystem nach einem der Ansprüche 12 oder 13 transformiert, transfiziert oder infiziert worden ist.

16. Wirtszelle nach den Ansprüchen 14 oder 15, wobei die genannte Zelle aus einer Säugetierzelle, einer Vogelzelle, einer Insektenzelle und einer Hefe ausgewählt wird.

17. Vorgang zur Produktion von chimären, leeren Kapsiden des Virus der infektiösen Bursitis (IBDV) nach einem der Ansprüche 1 bis 8, welcher das Kultivieren einer Wirtszelle nach einem der Ansprüche 14 bis 16, und, falls gewünscht, das Wiederherstellen der genannten chimären, leeren IBDV-Kapsiden umfasst.

18. Vorgang nach Anspruch 17, bei dem die genannte Wirtszelle eine Insektenzelle ist, welcher folgende Schritte umfasst;
a) Aufbereiten eines aus (I) und (II) ausgewählten Expressionssystems, bei dem:
- das Expressionssystem (I) aus einem rekombinanten Bakulovirus besteht, das ein Genkonstrukt nach Anspruch 11 enthält; und
- das Expressionssystem (II) aus einem ersten rekombinanten Bakulovirus besteht, das ein Genkonstrukt enthält, welches das IBDV-pVP2-Protein codiert, und einem zweiten rekombinanten Bakulovirus, das ein Genkonstrukt enthält, welches eine Nukleinsäure umfasst, welche (i) eine den offenen Leserahmen entsprechend dem IBDV-VP3-Protein umfassende Nukleotidsequenz umfasst, und (ii) eine Nukleotidsequenz, welche den offenen Leserahmen eines heterologen Polypeptids umfasst, der ein interessierendes Polypeptid umfasst, bei dem in dem resultierenden Fusionsprotein das genannte heterologe Polypeptid mit dem aminoterminalen Bereich des IVBDV-VP3 verbunden ist;
b) Infizieren von Insektenzellen mit dem genannten in Schritt a) aufbereiteten Expressionssystem;
c) Kultivieren der in Schritt b) erhaltenen infizierten Insektenzellen unter Bedingungen, welche die Expression von rekombinanten Proteinen und ihrer Anordnung erlauben, um chimäre, leere IBDV-Kapside zu bilden; und
d) falls gewünscht, Isolieren und wahlweise Aufreinigen der chimären, leeren IBDV-Kapside.

19. Vorgang nach Anspruch 17, bei dem die genannte Wirtszelle eine Hefe ist, welche folgende Schritte umfasst:
a) Aufbereiten eines aus einem ein Genkonstrukt nach Anspruch 11 enthaltenden Plasmid bestehenden Expressionssystem;
b) Transformieren von Hefezellen mit dem genannten in Schritt a) aufbereiteten Expressionssystem;
c) Kultivieren der in Schritt b) erhaltenen transformierten Hefen unter Bedingungen, welche die Expression von rekombinanten Proteinen und ihrer Anordnung erlauben, um chimäre, leere IBDV-Kapside zu bilden; und
d) falls gewünscht, Isolieren und wahlweise Aufreinigen der chimären, leeren IBDV-Kapside.

20. Verwendung eines Genexpressionssystems nach einem der Ansprüche 12 oder 13 zum Produzieren chimärer, leerer IBDV-Kapside nach einem der Ansprüche 1 bis 8.

21. Verwendung chimärer, leerer Kapside des Virus der infektiösen Bursitis (IBDV) nach einem der Ansprüche 1 bis 8 bei der Herstellung eines Medikaments.

22. Verwendung nach Anspruch 21, bei der das genannte Medikament ein Impfstoff ist.

23. Verwendung nach Anspruch 21, bei der das genannte Medikament ein Gentherapievektor ist.

24. Impfstoff, welcher einen therapeutisch effizienten Betrag an chimären, leeren Kapsiden des Virus der infektiösen Bursitis (IBBV) nach einem der Ansprüche 1 bis 8 umfasst, wahlweise zusammen mit einem oder mehr pharmazeutisch akzeptablen Zusatzstoffen und/oder Trägerstoffen.

25. Impfstoff nach Anspruch 24, welcher für den gleichzeitigen Schutz von Tieren oder Menschen vor durch zwei oder mehr krankheitsverursachende Infektionserreger verursachten Infektionen nützlich ist.

26. Gentherapievektor, welcher ein chimäres, leeres Kapsid des Virus der infektiösen Bursitis (IBDV) nach einem der Ansprüche 1 bis 8 umfasst.

## Revendications

1. Capside chimère vide du virus de la bursite infectieuse (VBI), **caractérisée en ce qu'**elle est constituée par l'assemblage de (i) protéines pVP2 de VBI et (ii) de protéines de fusions comprenant une région A constituée par la protéine VP3 de VBI unie à une région B constituée par un polypeptide hétérologue comprenant un polypeptide d'intérêt, dans laquelle ladite région B est unie à la région amino-terminale de VP3 de VSI.

2. Capside selon la revendication 1, dans laquelle ledit polypeptide d'intérêt est un polypeptide utile à des fins de vaccination, de thérapie ou de diagnose.

3. Capside selon la revendication 1, dans laquelle ladite région B comprend un simple polypeptide d'intérêt.

4. Capside selon la revendication 1, dans laquelle ladite région B comprend deux ou plusieurs polypeptides d'intérêt.

5. Capside selon la revendication 1, dans laquelle ladite protéine de fusion comprend une région A unie à une simple région B.

6. Capside selon la revendication 1, dans laquelle ladite protéine de fusion comprend une région A unie à deux régions B, identiques ou différentes, une des quelles unie à la région amino-terminale de VP3 présente dans la région A, et l'autre à la région carboxi-terminale de la VP3 présente dans la région A.

7. Capside selon la revendication 6, dans laquelle lesdites régions B contiennent plus d'un polypeptide d'intérêt identiques ou différents les uns des autres.

8. Capside selon la revendication 1, dans laquelle ladite protéine de fusion comprend en outre, un polypeptide lieur situé entre lesdites régions A et B.

9. Acide nucléique comprenant une séquence de nucléotides codant pour les capsides chimères vides des revendications 1 à 8, qui comprend les séquences de nucléotides codant pour la protéine de fusion définie dans l'une quelconque des revendications 1 à 8, comprenant en outre (iii) une séquence de nucléotides comprenant le cadre de lecture ouvert correspondant à la protéine pVP2 de VBI.

10. Acide nucléique selon la revendication 9 dans lequel la séquence de nucléotides codant pour la protéine de fusion, comprend (i) une séquence de nucléotides comprenant le cadre de lecture ouvert correspondant à la protéine VP3 de VBI et (ii) une séquence de nucléotides comprenant le cadre de lecture ouvert d'un polypeptide hétérologue comprenant un polypeptide d'intérêt, dans lequel dans la protéine de fusion résultante ledit polypeptide hétérologue est uni à la région amino-terminale de VP3 de VBI.

11. Construction génique comprenant un acide nucléique selon la revendication 9 ou 10.

12. Système d'expression sélectionné parmi :
a) un système d'expression comprenant une première construction génique comprenant un acide nucléique qui comprend (i) une séquence de nucléotides comprenant le cadre de lecture ouvert correspondant à la protéine VP3 de VBI et (ii) une séquence de nucléotides comprenant le cadre de lecture ouvert d'un polypeptide hétérologue comprenant un polypeptide d'intérêt, dans lequel dans la protéine de fusion résultante ledit polypeptide hétérologue est uni à la région amino-terminale de VP3 de VBI, uni opérationnellement à des éléments de contrôlé de la transcription, et optionnellement de la traduction, et une deuxième construction génique, opérationnellement uni à des éléments de contrôle de la transcription, et optionnellement de la traduction ; ladite deuxième construction génique comprenant une séquence de nucléotides comprenant le cade de lecture ouvert correspondant à la protéine pVP2 de VBI ; et
b) un système d'expression comprenant une construction génique selon la revendication 11, uni opérationnellement à des éléments de contrôlé de la transcription, et optionnellement de la traduction.

13. Système d'expression selon la revendication 12, ledit système d'expression étant sélectionné parmi les plasmides, bacmides, les chromosomes artificiels de levure (YAC), les chromosomes artificiels de bactéries (BAC), les chromosomes artificiels basés sur les bactériophages PI (PAC), les cosmides, ou les virus, qui contiennent optionnellement une origine de réplication hétérologue.

14. Cellule hôte contenant un acide nucléique selon l'une quelconque des revendications 9 ou 10 ou une construction génique selon la revendication 11, ou un système d'expression selon l'une quelconque des revendications 12 ou 13.

15. Cellule hôte, ladite cellule ayant été transformée, transfectée ou infectée par un système d'expression selon l'une quelconque des revendications 12 ou 13.

16. Cellule hôte selon les revendications 14 ou 15, ladite cellule ayant été sélectionnée à partir d'une cellule mammifère, une cellule aviaire, une cellule d'insecte et une levure.

17. Procédé pour la production de capsides chimères vides du virus de la bursite infectieuse (VBI) selon l'une quelconque des revendications 1 à 8, comprenant la culture d'une cellule hôte selon l'une quelconque des revendications 14 ou 16, et si l'on le souhaite, la récupération desdits capsides chimères vides de VBI.

18. Procédé selon la revendication 17, dans lequel ladite cellule hôte est une cellule d'insecte, comprenant les étapes de :
a) préparer un système d'expression sélectionne parmi (I) et (II) ;
- le système d'expression (I) est constitué d'un baculovirus recombinant contenant une construction génique selon la revendication 11 ; et
- le système d'expression (II) est constitué d'un premier baculovirus recombinant contenant une construction génique codant pour la protéine pVP2 de VBI, et un deuxième baculovirus recombinant contenant une construction génique comprenant un acide nucléique qui comprend (i) une séquence de nucléotides comprenant le cadre de lecture ouvert correspondant à la protéine VP3 de VBI et (ii) une séquence de nucléotides comprenant le cadre de lecture ouvert d'un polypeptide hétérologue comprenant un polypeptide d'intérêt, dans lequel dans la protéine de fusion résultante ledit polypeptide hétérologue est uni à la région amino-terminale de VP3 de VBI.
b) infecter des cellules d'insecte avec ledit système d'expression préparé dans l'étape a) ;
c) cultiver les cellules d'insecte infectées obtenues dans l'étape b) dans des conditions permettant l'expression de protéines recombinantes et leur assemblage pour former des capsides chimères vides de VBI, et ;
d) si l'on le souhaite, isoler et optionnellement purifier les capsides chimères vides de VBI.

19. Procédé selon la revendication 17, dans lequel ladite cellule hôte est une levure, comprenant les étapes de :
a) préparer un système d'expression constitué d'un plasmide contenant une construction génique selon la revendication 11 ;
b) transformer des cellules de levure avec ledit système d'expression préparé dans l'étape a);
c) cultiver les levures transformées obtenues dans l'étape b) dans des conditions permettant l'expression de protéines recombinantes et leur assemblage pour former des capsides chimères vides de VBI, et ;
d) si l'on le souhaite, isoler et optionnellement purifier les capsides chimères vides de VBI.

20. Utilisation d'un système d'expression génique selon l'une quelconque des revendications 12 ou 13 pour produire des capsides chimères vides de VBI selon l'une quelconque des revendications 1 à 8.

21. Utilisation de capsides chimères vides du virus de la bursite infectieuse (VBI) selon l'une quelconque des revendications 1 à 8 dans l'élaboration d'un médicament.

22. Utilisation selon la revendication 21, dans laquelle ledit médicament est un vaccin.

23. Utilisation selon la revendication 21, dans lequel ledit médicament est un vecteur de thérapie génique.

24. Vaccin comprenant une quantité thérapeutiquement effective de capsides chimères vides du virus de la bursite infectieuse (VBI) selon l'une quelconque des revendications 1 à 8, optionnellement avec un ou plusieurs adjuvants et/ou véhicules pharmaceutiquement acceptables.

25. Vaccin selon la revendication 24, utile pour protéger simultanément les animaux ou les hommes contre l'infection causée par deux ou plusieurs agents infectieux causant des maladies.

26. Vecteur de thérapie génique comprenant une capside chimère vide du virus de la bursite infectieuse (VBI) selon l'une quelconque des revendications 1 à 8.


### SEQUENCE LISTING

<110> CONSEJO SUPERIOR DE INVESTIGACIONES CIENTIFICAS
   <110> BIONOSTRA, S.L.
<120> CHIMERIC EMPTY CAPSIDS OF THE INFECTIOUS BURSAL DISEASE VIRUS
   (IBDV), OBTAINMENT PROCESS AND APPLICATIONS
<130> P1391PC
<150> ES P200400120
   <151> 2004-01-21 (January 21, 2004)
<160> 14
<170> PatentIn version 3.1
<210> 1
   <211> 35
   <212> DNA
   <213> Artificial sequence
<220> Synthetic DNA
   <223> Oligo I primer
<400> 1
   gcgcagatct atgacaaacc tgtcagatca aaccc 35
<210> 2
   <211> 34
   <212> DNA
   <213> Artificial sequence
<220> Synthetic DNA
   <223> Oligo II primer
<400> 2
   gcgcaagctt aggcgagagt cagctgcctt atgc 34
<210> 3
   <211> 7595
   <212> DNA
   <213> Artificial sequence
<220>
   <223> Plasmid pFBD/pVP2-his-VP3
<220>
   <221> promoter
   <222> (157)..(285)
   <223> Promoter ppolh
<220>
   <221> CDS
   <222> (291)..(1289)
   <223> pVP2 ORF
<220>
   <221> promoter
   <222> (7443)..(7503)
   <223> Promoter p10
<400> 3
   gggtgatcaa gtcttcgtcg agtgattgta aataaaatgt aatttacagt atagtatttt    60
   aattaatata caaatgattt gataataatt cttatttaac tataatatat tgtgttgggt   120
   tgaattaaag gtccgtatac tccggaatat taatagatca tggagataat taaaatgata   180
   accatctcgc aaataaataa gtattttact gttttcgtaa cagttttgta ataaaaaaac   240
   ctataaatat tccggattat tcataccgtc ccaccatcgg gcgcggatct atg aca      296
                                                          Met Thr
                                                          1
   aac ctg tca gat caa acc cag cag att gtt ccg ttc ata cgg agc ctt     344
   Asn Leu Ser Asp Gln Thr Gln Gln Ile Val Pro Phe Ile Arg Ser Leu
           5                   10                  15
   ctg atg cca aca acc gga ccg gcg tcc att ccg gac gac acc ctg gag     392
   Leu Met Pro Thr Thr Gly Pro Ala Ser Ile Pro Asp Asp Thr Leu Glu
       20                  25                  30
   aag cac act ctc agg tca gag acc tcg acc tac aat ttg act gtg ggg     440
   Lys His Thr Leu Arg Ser Glu Thr Ser Thr Tyr Asn Leu Thr Val Gly
   35                  40                  45                  50
   gac aca ggg tca ggg cta att gtc ttt ttc cct gga ttc cct ggc tca     488
   Asp Thr Gly Ser Gly Leu Ile Val Phe Phe Pro Gly Phe Pro Gly Ser
                   55                  60                  65
   att gtg ggt gct cac tac aca ctg cag ggc aat ggg aac tac aag ttc     536
   Ile Val Gly Ala His Tyr Thr Leu Gln Gly Asn Gly Asn Tyr Lys Phe
               70                  75                  80
   gat cag atg ctc ctg act gcc cag aac cta ccg gcc agt tac aac tac     584
   Asp Gln Met Leu Leu Thr Ala Gln Asn Leu Pro Ala Ser Tyr Asn Tyr
           85                  90                  95
   tgc agg cta gtg agt cgg agt ctc aca gtg agg tca agc aca ctt cct     632
   Cys Arg Leu Val Ser Arg Ser Leu Thr Val Arg Ser Ser Thr Leu Pro
       100                 105                 110
   ggt ggc gtt tat gca cta aac ggc acc ata aac gcc gtg acc ttc caa     680
   Gly Gly Val Tyr Ala Leu Asn Gly Thr Ile Asn Ala Val Thr Phe Gln
   115                 120                 125                 130
   gga agc ctg agt gaa ctg aca gat gtt agc tac aat ggg ttg atg tct     728
   Gly Ser Leu Ser Glu Leu Thr Asp Val Ser Tyr Asn Gly Leu Met Ser
                   135                 140                 145
   gca aca gcc aac atc aac gac aaa att ggg aac gtc cta gta ggg gaa     776
   Ala Thr Ala Asn Ile Asn Asp Lys Ile Gly Asn Val Leu Val Gly Glu
               150                 155                 160
   ggg gtc acc gtc ctc agc tta ccc aca tca tat gat ctt ggg tat gtg      824
   Gly Val Thr Val Leu Ser Leu Pro Thr Ser Tyr Asp Leu Gly Tyr Val
           165                 170                 175
   agg ctt ggt gac ccc att ccc gca ata ggg ctt gac cca aaa atg gta      872
   Arg Leu Gly Asp Pro Ile Pro Ala Ile Gly Leu Asp Pro Lys Met Val
       180                 185                 190
   gcc aca tgt gac agc agt gac agg ccc aga gtc tac acc ata act gca      920
   Ala Thr Cys Asp Ser Ser Asp Arg Pro Arg Val Tyr Thr Ile Thr Ala
   195                 200                 205                 210
   gcc gat gat tac caa ttc tca tca cag tac caa cca ggt ggg gta aca      968
   Ala Asp Asp Tyr Gln Phe Ser Ser Gln Tyr Gln Pro Gly Gly Val Thr
                   215                 220                 225
   atc aca ctg ttc tca gcc aac att gat gcc atc aca agc ctc agc gtt     1016
   Ile Thr Leu Phe Ser Ala Asn Ile Asp Ala Ile Thr Ser Leu Ser Val
               230                 235                 240
   ggg gga gag ctc gtg ttt cga aca agc gtc cac ggc ctt gta ctg ggc     1064
   Gly Gly Glu Leu Val Phe Arg Thr Ser Val His Gly Leu Val Leu Gly
           245                 250                 255
   gcc acc atc tac ctc ata ggc ttt gat ggg aca acg gta atc acc agg     1112
   Ala Thr Ile Tyr Leu Ile Gly Phe Asp Gly Thr Thr Val Ile Thr Arg
       260                 265                 270
   gct gtg gcc gca aac aat ggg ctg acg acc ggc acc gac aac ctt atg     1160
   Ala Val Ala Ala Asn Asn Gly Leu Thr Thr Gly Thr Asp Asn Leu Met
   275                 280                 285                 290
   cca ttc aat ctt gtg att cca aca aac gag ata acc cag cca atc aca     1208
   Pro Phe Asn Leu Val Ile Pro Thr Asn Glu Ile Thr Gln Pro Ile Thr
                   295                 300                 305
   tcc atc aaa ctg gag ata gtg acc tcc aaa agt ggt ggt cag gca ggg     1256
   Ser Ile Lys Leu Glu Ile Val Thr Ser Lys Ser Gly Gly Gln Ala Gly
               310                 315                 320
   gat cag atg tca tgg tcg gca aga ggg agc cta gcagtgacga tccatggtgg   1309
   Asp Gln Met Ser Trp Ser Ala Arg Gly Ser Leu
           325                 330
   caactatcca ggggccctcc gtcccgtcac gctagtggcc tacgaaagag tggcaacagg   1369
   atccgtcgtt acggtcgctg gggtgagcaa cttcgagctg atcccaaatc ctgaactagc   1429
   aaagaacctg gttacagaat acggccgatt tgacccagga gccatgaact acacaaaatt   1489
   gatactgagt gagagggacc gtcttggcat caagaccgtc tggccaacaa gggagtacac   1549
   tgactttcgt gaatacttca tggaggtggc cgacctcaac tctcccctga agattgcagg   1609
   agcattcggc ttcaaagaca taatccgggc cataaggagg atagctgtgc cggtggtctc   1669
   cacattgttc ccacctgccg ctcccctagc ccatgcaatt ggggaaggtg tagactacct   1729
   gctgggcgat gaggcccagg ccgcttcagg aactgctcga gccgcgtcag gaaaagcaag   1789
   agctgcctca ggccgcataa ggcagctgac tctcgcctaa gcttgtcgag aagtactaga   1849
   ggatcataat cagccatacc acatttgtag aggttttact tgctttaaaa aacctcccac   1909
   acctccccct gaacctgaaa cataaaatga atgcaattgt tgttgttaac ttgtttattg   1969
   cagcttataa tggttacaaa taaagcaata gcatcacaaa tttcacaaat aaagcatttt   2029
   tttcactgca ttctagttgt ggtttgtcca aactcatcaa tgtatcttat catgtctgga   2089
   tctgatcact gcttgagcct aggagatccg aaccagataa gtgaaatcta gttccaaact   2149
   attttgtcat ttttaatttt cgtattagct tacgacgcta cacccagttc ccatctattt   2209
   tgtcactctt ccctaaataa tccttaaaaa ctccatttcc acccctccca gttcccaact   2269
   attttgtccg cccacagcgg ggcatttttc ttcctgttat gtttttaatc aaacatcctg   2329
   ccaactccat gtgacaaacc gtcatcttcg gctacttttt ctctgtcaca gaatgaaaat   2389
   ttttctgtca tctcttcgtt attaatgttt gtaattgact gaatatcaac gcttatttgc   2449
   agcctgaatg gcgaatggga cgcgccctgt agcggcgcat taagcgcggc gggtgtggtg   2509
   gttacgcgca gcgtgaccgc tacacttgcc agcgccctag cgcccgctcc tttcgctttc   2569
   ttcccttcct ttctcgccac gttcgccggc tttccccgtc aagctctaaa tcgggggctc   2629
   cctttagggt tccgatttag tgctttacgg cacctcgacc ccaaaaaact tgattagggt   2689
   gatggttcac gtagtgggcc atcgccctga tagacggttt ttcgcccttt gacgttggag   2749
   tccacgttct ttaatagtgg actcttgttc caaactggaa caacactcaa ccctatctcg   2809
   gtctattctt ttgatttata agggattttg ccgatttcgg cctattggtt aaaaaatgag   2869
   ctgatttaac aaaaatttaa cgcgaatttt aacaaaatat taacgtttac aatttcaggt   2929
   ggcacttttc ggggaaatgt gcgcggaacc cctatttgtt tatttttcta aatacattca   2989
   aatatgtatc cgctcatgag acaataaccc tgataaatgc ttcaataata ttgaaaaagg   3049
   aagagtatga gtattcaaca tttccgtgtc gcccttattc ccttttttgc ggcattttgc   3109
   cttcctgttt ttgctcaccc agaaacgctg gtgaaagtaa aagatgctga agatcagttg   3169
   ggtgcacgag tgggttacat cgaactggat ctcaacagcg gtaagatcct tgagagtttt   3229
   cgccccgaag aacgttttcc aatgatgagc acttttaaag ttctgctatg tggcgcggta   3289
   ttatcccgta ttgacgccgg gcaagagcaa ctcggtcgcc gcatacacta ttctcagaat   3349
   gacttggttg agtactcacc agtcacagaa aagcatctta cggatggcat gacagtaaga   3409
   gaattatgca gtgctgccat aaccatgagt gataacactg cggccaactt acttctgaca   3469
   acgatcggag gaccgaagga gctaaccgct tttttgcaca acatggggga tcatgtaact   3529
   cgccttgatc gttgggaacc ggagctgaat gaagccatac caaacgacga gcgtgacacc   3589
   acgatgcctg tagcaatggc aacaacgttg cgcaaactat taactggcga actacttact 3649
   ctagcttccc ggcaacaatt aatagactgg atggaggcgg ataaagttgc aggaccactt 3709
   ctgcgctcgg cccttccggc tggctggttt attgctgata aatctggagc cggtgagcgt 3769
   gggtctcgcg gtatcattgc agcactgggg ccagatggta agccctcccg tatcgtagtt 3829
   atctacacga cggggagtca ggcaactatg gatgaacgaa atagacagat cgctgagata 3889
   ggtgcctcac tgattaagca ttggtaactg tcagaccaag tttactcata tatactttag 3949
   attgatttaa aacttcattt ttaatttaaa aggatctagg tgaagatcct ttttgataat 4009
   ctcatgacca aaatccctta acgtgagttt tcgttccact gagcgtcaga ccccgtagaa 4069
   aagatcaaag gatcttcttg agatcctttt tttctgcgcg taatctgctg cttgcaaaca 4129
   aaaaaaccac cgctaccagc ggtggtttgt ttgccggatc aagagctacc aactcttttt 4189
   ccgaaggtaa ctggcttcag cagagcgcag ataccaaata ctgtccttct agtgtagccg 4249
   tagttaggcc accacttcaa gaactctgta gcaccgccta catacctcgc tctgctaatc 4309
   ctgttaccag tggctgctgc cagtggcgat aagtcgtgtc ttaccgggtt ggactcaaga 4369
   cgatagttac cggataaggc gcagcggtcg ggctgaacgg ggggttcgtg cacacagccc 4429
   agcttggagc gaacgaccta caccgaactg agatacctac agcgtgagca ttgagaaagc 4489
   gccacgcttc ccgaagggag aaaggcggac aggtatccgg taagcggcag ggtcggaaca 4549
   ggagagcgca cgagggagct tccaggggga aacgcctggt atctttatag tcctgtcggg 4609
   tttcgccacc tctgacttga gcgtcgattt ttgtgatgct cgtcaggggg gcggagccta 4669
   tggaaaaacg ccagcaacgc ggccttttta cggttcctgg ccttttgctg gccttttgct 4729
   cacatgttct ttcctgcgtt atcccctgat tctgtggata accgtattac cgcctttgag 4789
   tgagctgata ccgctcgccg cagccgaacg accgagcgca gcgagtcagt gagcgaggaa 4849
   gcggaagagc gcctgatgcg gtattttctc cttacgcatc tgtgcggtat ttcacaccgc 4909
   agaccagccg cgtaacctgg caaaatcggt tacggttgag taataaatgg atgccctgcg 4969
   taagcgggtg tgggcggaca ataaagtctt aaactgaaca aaatagatct aaactatgac 5029
   aataaagtct taaactagac agaatagttg taaactgaaa tcagtccagt tatgctgtga 5089
   aaaagcatac tggacttttg ttatggctaa agcaaactct tcattttctg aagtgcaaat 5149
   tgcccgtcgt attaaagagg ggcgtggcca agggcatggt aaagactata ttcgcggcgt 5209
   tgtgacaatt taccgaacaa ctccgcggcc gggaagccga tctcggcttg aacgaattgt 5269
   taggtggcgg tacttgggtc gatatcaaag tgcatcactt cttcccgtat gcccaacttt 5329
   gtatagagag ccactgcggg atcgtcaccg taatctgctt gcacgtagat cacataagca 5389
   ccaagcgcgt tggcctcatg cttgaggaga ttgatgagcg cggtggcaat gccctgcctc 5449
   cggtgctcgc cggagactgc gagatcatag atatagatct cactacgcgg ctgctcaaac 5509
   ctgggcagaa cgtaagccgc gagagcgcca acaaccgctt cttggtcgaa ggcagcaagc 5569
   gcgatgaatg tcttactacg gagcaagttc ccgaggtaat cggagtccgg ctgatgttgg 5629
   gagtaggtgg ctacgtctcc gaactcacga ccgaaaagat caagagcagc ccgcatggat 5689
   ttgacttggt cagggccgag cctacatgtg cgaatgatgc ccatacttga gccacctaac 5749
   tttgttttag ggcgactgcc ctgctgcgta acatcgttgc tgctgcgtaa catcgttgct 5809
   gctccataac atcaaacatc gacccacggc gtaacgcgct tgctgcttgg atgcccgagg 5869
   catagactgt acaaaaaaac agtcataaca agccatgaaa accgccactg cgccgttacc 5929
   accgctgcgt tcggtcaagg ttctggacca gttgcgtgag cgcatacgct acttgcatta 5989
   cagtttacga accgaacagg cttatgtcaa ctgggttcgt gccttcatcc gtttccacgg 6049
   tgtgcgtcac ccggcaacct tgggcagcag cgaagtcgag gcatttctgt cctggctggc 6109
   gaacgagcgc aaggtttcgg tctccacgca tcgtcaggca ttggcggcct tgctgttctt 6169
   ctacggcaag gtgctgtgca cggatctgcc ctggcttcag gagatcggta gacctcggcc 6229
   gtcgcggcgc ttgccggtgg tgctgacccc ggatgaagtg gttcgcatcc tcggttttct 6289
   ggaaggcgag catcgtttgt tcgcccagga ctctagctat agttctagtg gttggcctac 6349
   gtacccgtag tggctatggc agggcttgcc gccccgacgt tggctgcgag ccctgggcct 6409
   tcacccgaac ttgggggttg gggtggggaa aaggaagaaa cgcgggcgta ttggtcccaa 6469
   tggggtctcg gtggggtatc gacagagtgc cagccctggg accgaacccc gcgtttatga 6529
   acaaacgacc caacacccgt gcgttttatt ctgtcttttt attgccgtca tagcgcgggt 6589
   tccttccggt attgtctcct tccgtgtttc agttagcctc ccccatctcc cggtaccgca 6649
   tgcctcgaga ctgcaggctc tagattcgaa agcggccgcg actagtgagc tcgtcgacgt 6709
   aggcctttga attccggatc ctcactcaag gtcctcatca gagacggtcc tgatccagcg 6769
   gcccagccga ccagggggtc tctgtgttgg agcattgggt tttggcttgg gctttggtag 6829
   agcccgcctg ggattgcgat gcttcatctc catcgcagtc aagagcagat ctttcatctg 6889
   ttcttggttt gggccacgtc catggttgat ttcatagact ttggcaactt cgtctatgaa 6949
   agcttggggt ggctctgcct gtcctggagc cccgtagatc gacgtagctg cccttaggat 7009
   ttgttcttct gatgccaacc ggctcttctc tgcatgcacg tagtctagat agtcctcgtt 7069
   tgggtccggt atttctcgtt tgttctgcca gtactttacc tggcctgggc ttggccctcg 7129
   gtgcccattg agtgctaccc attctggtgt tgcaaagtag atgcccatgg tctccatctt 7189
   ctttgagatc cgtgtgtctt tttccctctg tgcttcctct ggtgtggggc cccgagcctc 7249
   cactccgtag cctgctgtcc cgtacttggc cctttgcgac ttgctgcctg cttgtggtgc 7309
   gtttgcaaga aaatttcgca tccgatgggc gttcgggtcg ctgagtgcga agttggccat 7369
   gtcagtcaca atcccattct cttccagcca catgaacaca ctgagtgcag attggaatag 7429
   tgggtccacg ttggctgctg cttccattgc tctgacggca ctctcgagtt cgggggtctc 7489
   tttgaactct gatgcagcca tggcgccctg aaaatacagg ttttcggtcg ttgggatatc 7549
   gtaatcgtga tggtgatggt gatggtagta cgacatggtt tcggac 7595
<210> 4
   <211> 333
   <212> PRT
   <213> Artificial sequence
<220>
   <223> pVP2-his-VP3 protein
<400> 4
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu
                   325                 330
<210> 5
   <211> 35
   <212> DNA
   <213> Artificial sequence
<220> Synthetic DNA
   <223> Oligo III primer
<400> 5
   gcgcagatct atgacaaacc tgtcagatca aaccc 35
<210> 6
   <211> 34
   <212> DNA
   <213> Artificial sequence
<220> Synthetic DNA
   <223> Oligo IV primer
<400> 6
   gcgcaagctt aggcgagagt cagctgcctt atgc 34
<210> 7
   <211> 9600
   <212> DNA
   <213> Artificial sequence
<220>
   <223> Plasmid pESCURA/pVP2-VP3-GFP
<220>
   <221> promoter
   <222> (5649).. (5859)
   <223> Promoter 1 (pVP2)
<220>
   <221> promoter
   <222> (7402)..(8080)
   <223> Promoter 2 (VP3-GFP)
<220>
   <221> CDS
   <222> (8086)..(9597)
   <223> VP3-GFP ORF
<400> 7
   ggccgcacta gtatcgatgg attacaagga tgacgacgat aagatctgag ctcttaatta     60
   acaattcttc gccagaggtt tggtcaagtc tccaatcaag gttgtcggct tgtctacctt    120
   gccagaaatt tacgaaaaga tggaaaaggg tcaaatcgtt ggtagatacg ttgttgacac    180
   ttctaaataa gcgaatttct tatgatttat gatttttatt attaaataag ttataaaaaa    240
   aataagtgta tacaaatttt aaagtgactc ttaggtttta aaacgaaaat tcttattctt    300
   gagtaactct ttcctgtagg tcaggttgct ttctcaggta tagcatgagg tcgctccaat    360
   tcagctgcat taatgaatcg gccaacgcgc ggggagaggc ggtttgcgta ttgggcgctc    420
   ttccgcttcc tcgctcactg actcgctgcg ctcggtcgtt cggctgcggc gagcggtatc    480
   agctcactca aaggcggtaa tacggttatc cacagaatca ggggataacg caggaaagaa    540
   catgtgagca aaaggccagc aaaaggccag gaaccgtaaa aaggccgcgt tgctggcgtt    600
   tttccatagg ctccgccccc ctgacgagca tcacaaaaat cgacgctcaa gtcagaggtg    660
   gcgaaacccg acaggactat aaagatacca ggcgtttccc cctggaagct ccctcgtgcg    720
   ctctcctgtt ccgaccctgc cgcttaccgg atacctgtcc gcctttctcc cttcgggaag    780
   cgtggcgctt tctcatagct cacgctgtag gtatctcagt tcggtgtagg tcgttcgctc    840
   caagctgggc tgtgtgcacg aaccccccgt tcagcccgac cgctgcgcct tatccggtaa    900
   ctatcgtctt gagtccaacc cggtaagaca cgacttatcg ccactggcag cagccactgg    960
   taacaggatt agcagagcga ggtatgtagg cggtgctaca gagttcttga agtggtggcc   1020
   taactacggc tacactagaa ggacagtatt tggtatctgc gctctgctga agccagttac   1080
   cttcggaaaa agagttggta gctcttgatc cggcaaacaa accaccgctg gtagcggtgg   1140
   tttttttgtt tgcaagcagc agattacgcg cagaaaaaaa ggatctcaag aagatccttt   1200
   gatcttttct acggggtctg acgctcagtg gaacgaaaac tcacgttaag ggattttggt   1260
   catgagatta tcaaaaagga tcttcaccta gatcctttta aattaaaaat gaagttttaa 1320
   atcaatctaa agtatatatg agtaaacttg gtctgacagt taccaatgct taatcagtga 1380
   ggcacctatc tcagcgatct gtctatttcg ttcatccata gttgcctgac tccccgtcgt 1440
   gtagataact acgatacggg agggcttacc atctggcccc agtgctgcaa tgataccgcg 1500
   agacccacgc tcaccggctc cagatttatc agcaataaac cagccagccg gaagggccga 1560
   gcgcagaagt ggtcctgcaa ctttatccgc ctccatccag tctattaatt gttgccggga 1620
   agctagagta agtagttcgc cagttaatag tttgcgcaac gttgttgcca ttgctacagg 1680
   catcgtggtg tcacgctcgt cgtttggtat ggcttcattc agctccggtt cccaacgatc 1740
   aaggcgagtt acatgatccc ccatgttgtg caaaaaagcg gttagctcct tcggtcctcc 1800
   gatcgttgtc agaagtaagt tggccgcagt gttatcactc atggttatgg cagcactgca 1860
   taattctctt actgtcatgc catccgtaag atgcttttct gtgactggtg agtactcaac 1920
   caagtcattc tgagaatagt gtatgcggcg accgagttgc tcttgcccgg cgtcaatacg 1980
   ggataatacc gcgccacata gcagaacttt aaaagtgctc atcattggaa aacgttcttc 2040
   ggggcgaaaa ctctcaagga tcttaccgct gttgagatcc agttcgatgt aacccactcg 2100
   tgcacccaac tgatcttcag catcttttac tttcaccagc gtttctgggt gagcaaaaac 2160
   aggaaggcaa aatgccgcaa aaaagggaat aagggcgaca cggaaatgtt gaatactcat 2220
   actcttcctt tttcaatatt attgaagcat ttatcagggt tattgtctca tgagcggata 2280
   catatttgaa tgtatttaga aaaataaaca aataggggtt ccgcgcacat ttccccgaaa 2340
   agtgccacct gaacgaagca tctgtgcttc attttgtaga acaaaaatgc aacgcgagag 2400
   cgctaatttt tcaaacaaag aatctgagct gcatttttac agaacagaaa tgcaacgcga 2460
   aagcgctatt ttaccaacga agaatctgtg cttcattttt gtaaaacaaa aatgcaacgc 2520
   gagagcgcta atttttcaaa caaagaatct gagctgcatt tttacagaac agaaatgcaa 2580
   cgcgagagcg ctattttacc aacaaagaat ctatacttct tttttgttct acaaaaatgc 2640
   atcccgagag cgctattttt ctaacaaagc atcttagatt actttttttc tcctttgtgc 2700
   gctctataat gcagtctctt gataactttt tgcactgtag gtccgttaag gttagaagaa 2760
   ggctactttg gtgtctattt tctcttccat aaaaaaagcc tgactccact tcccgcgttt 2820
   actgattact agcgaagctg cgggtgcatt ttttcaagat aaaggcatcc ccgattatat 2880
   tctataccga tgtggattgc gcatactttg tgaacagaaa gtgatagcgt tgatgattct 2940
   tcattggtca gaaaattatg aacggtttct tctattttgt ctctatatac tacgtatagg 3000
   aaatgtttac attttcgtat tgttttcgat tcactctatg aatagttctt actacaattt 3060
   ttttgtctaa agagtaatac tagagataaa cataaaaaat gtagaggtcg agtttagatg 3120
   caagttcaag gagcgaaagg tggatgggta ggttatatag ggatatagca cagagatata 3180
   tagcaaagag atacttttga gcaatgtttg tggaagcggt attcgcaata ttttagtagc 3240
   tcgttacagt ccggtgcgtt tttggttttt tgaaagtgcg tcttcagagc gcttttggtt 3300
   ttcaaaagcg ctctgaagtt cctatacttt ctagagaata ggaacttcgg aataggaact 3360
   tcaaagcgtt tccgaaaacg agcgcttccg aaaatgcaac gcgagctgcg cacatacagc 3420
   tcactgttca cgtcgcacct atatctgcgt gttgcctgta tatatatata catgagaaga 3480
   acggcatagt gcgtgtttat gcttaaatgc gtacttatat gcgtctattt atgtaggatg 3540
   aaaggtagtc tagtacctcc tgtgatatta tcccattcca tgcggggtat cgtatgcttc 3600
   cttcagcact accctttagc tgttctatat gctgccactc ctcaattgga ttagtctcat 3660
   ccttcaatgc tatcatttcc tttgatattg gatcatacta agaaaccatt attatcatga 3720
   cattaaccta taaaaatagg cgtatcacga ggccctttcg tctcgcgcgt ttcggtgatg 3780
   acggtgaaaa cctctgacac atgcagctcc cggagacggt cacagcttgt ctgtaagcgg 3840
   atgccgggag cagacaagcc cgtcagggcg cgtcagcggg tgttggcggg tgtcggggct 3900
   ggcttaacta tgcggcatca gagcagattg tactgagagt gcaccatacc acagcttttc 3960
   aattcaattc atcatttttt ttttattctt ttttttgatt tcggtttctt tgaaattttt 4020
   ttgattcggt aatctccgaa cagaaggaag aacgaaggaa ggagcacaga cttagattgg 4080
   tatatatacg catatgtagt gttgaagaaa catgaaattg cccagtattc ttaacccaac 4140
   tgcacagaac aaaaacctgc aggaaacgaa gataaatcat gtcgaaagct acatataagg 4200
   aacgtgctgc tactcatcct agtcctgttg ctgccaagct atttaatatc atgcacgaaa 4260
   agcaaacaaa cttgtgtgct tcattggatg ttcgtaccac caaggaatta ctggagttag 4320
   ttgaagcatt aggtcccaaa atttgtttac taaaaacaca tgtggatatc ttgactgatt 4380
   tttccatgga gggcacagtt aagccgctaa aggcattatc cgccaagtac aattttttac 4440
   tcttcgaaga cagaaaattt gctgacattg gtaatacagt caaattgcag tactctgcgg 4500
   gtgtatacag aatagcagaa tgggcagaca ttacgaatgc acacggtgtg gtgggcccag 4560
   gtattgttag cggtttgaag caggcggcag aagaagtaac aaaggaacct agaggccttt 4620
   tgatgttagc agaattgtca tgcaagggct ccctatctac tggagaatat actaagggta 4680
   ctgttgacat tgcgaagagc gacaaagatt ttgttatcgg ctttattgct caaagagaca 4740
   tgggtggaag agatgaaggt tacgattggt tgattatgac acccggtgtg ggtttagatg 4800
   acaagggaga cgcattgggt caacagtata gaaccgtgga tgatgtggtc tctacaggat 4860
   ctgacattat tattgttgga agaggactat ttgcaaaggg aagggatgct aaggtagagg 4920
   gtgaacgtta cagaaaagca ggctgggaag catatttgag aagatgcggc cagcaaaact 4980
   aaaaaactgt attataagta aatgcatgta tactaaactc acaaattaga gcttcaattt 5040
   aattatatca gttattaccc tatgcggtgt gaaataccgc acagatgcgt aaggagaaaa 5100
   taccgcatca ggaaattgta aacgttaata ttttgttaaa attcgcgtta aatttttgtt 5160
   aaatcagctc attttttaac caataggccg aaatcggcaa aatcccttat aaatcaaaag 5220
   aatagaccga gatagggttg agtgttgttc cagtttggaa caagagtcca ctattaaaga 5280
   acgtggactc caacgtcaaa gggcgaaaaa ccgtctatca gggcgatggc ccactacgtg 5340
   aaccatcacc ctaatcaagt tttttggggt cgaggtgccg taaagcacta aatcggaacc 5400
   ctaaagggag cccccgattt agagcttgac ggggaaagcc ggcgaacgtg gcgagaaagg 5460
   aagggaagaa agcgaaagga gcgggcgcta gggcgctggc aagtgtagcg gtcacgctgc 5520
   gcgtaaccac cacacccgcc gcgcttaatg cgccgctaca gggcgcgtcg cgccattcgc 5580
   cattcaggct gcgcaactgt tgggaagggc gatcggtgcg ggcctcttcg ctattacgcc 5640
   agctggatct tcgagcgtcc caaaaccttc tcaagcaagg ttttcagtat aatgttacat 5700
   gcgtacacgc gtctgtacag aaaaaaaaga aaaatttgaa atataaataa cgttcttaat 5760
   actaacataa ctataaaaaa ataaataggg acctagactt caggttgtct aactccttcc 5820
   ttttcggtta gagcggatct tagctagccg cggtaccaag cttaggcgag agtcagctgc 5880
   cttatgcggc ctgaggcagc tcttgctttt cctgacgcgg ctcgagcagt tcctgaagcg 5940
   gcctgggcct catcgcccag caggtagtct acaccttccc caattgcatg ggctagggga 6000
   gcggcaggtg ggaacaatgt ggagaccacc ggcacagcta tcctccttat ggcccggatt 6060
   atgtctttga agccgaatgc tcctgcaatc ttcaggggag agttgaggtc ggccacctcc 6120
   atgaagtatt cacgaaagtc agtgtactcc cttgttggcc agacggtctt gatgccaaga 6180
   cggtccctct cactcagtat caattttgtg tagttcatgg ctcctgggtc aaatcggccg 6240
   tattctgtaa ccaggttctt tgctagttca ggatttggga tcagctcgaa gttgctcacc 6300
   ccagcgaccg taacgacgga tcctgttgcc actctttcgt aggccactag cgtgacggga 6360
   cggagggccc ctggatagtt gccaccatgg atcgtcactg ctaggctccc tcttgccgac 6420
   catgacatct gatcccctgc ctgaccacca cttttggagg tcactatctc cagtttgatg 6480
   gatgtgattg gctgggttat ctcgtttgtt ggaatcacaa gattgaatgg cataaggttg 6540
   tcggtgccgg tcgtcagccc attgtttgcg gccacagccc tggtgattac cgttgtccca 6600
   tcaaagccta tgaggtagat ggtggcgccc agtacaaggc cgtggacgct tgttcgaaac 6660
   acgagctctc ccccaacgct gaggcttgtg atggcatcaa tgttggctga gaacagtgtg   6720
   attgttaccc cacctggttg gtactgtgat gagaattggt aatcatcggc tgcagttatg   6780
   gtgtagactc tgggcctgtc actgctgtca catgtggcta ccatttttgg gtcaagccct   6840
   attgcgggaa tggggtcacc aagcctcaca tacccaagat catatgatgt gggtaagctg   6900
   aggacggtga ccccttcccc tactaggacg ttcccaattt tgtcgttgat gttggctgtt   6960
   gcagacatca acccattgta gctaacatct gtcagttcac tcaggcttcc ttggaaggtc   7020
   acggcgttta tggtgccgtt tagtgcataa acgccaccag gaagtgtgct tgacctcact   7080
   gtgagactcc gactcactag cctgcagtag ttgtaactgg ccggtaggtt ctgggcagtc   7140
   aggagcatct gatcgaactt gtagttccca ttgccctgca gtgtgtagtg agcacccaca   7200
   attgagccag ggaatccagg gaaaaagaca attagccctg accctgtgtc ccccacagtc   7260
   aaattgtagg tcgaggtctc tgacctgaga gtgtgcttct ccagggtgtc gtccggaatg   7320
   gacgccggtc cggttgttgg catcagaagg ctccgtatga acggaacaat ctgctgggtt   7380
   tgatctgaca ggtttgtcat agatccgggg ttttttctcc ttgacgttaa agtatagagg   7440
   tatattaaca attttttgtt gatactttta ttacatttga ataagaagta atacaaaccg    7500
   aaaatgttga aagtattagt taaagtggtt atgcagtttt tgcatttata tatctgttaa   7560
   tagatcaaaa atcatcgctt cgctgattaa ttaccccaga aataaggcta aaaaactaat   7620
   cgcattatca tcctatggtt gttaatttga ttcgttcatt tgaaggtttg tggggccagg   7680
   ttactgccaa tttttcctct tcataaccat aaaagctagt attgtagaat ctttattgtt   7740
   cggagcagtg cggcgcgagg cacatctgcg tttcaggaac gcgaccggtg aagacgagga   7800
   cgcacggagg agagtcttcc ttcggagggc tgtcacccgc tcggcggctt ctaatccgta   7860
   cttcaatata gcaatgagca gttaagcgta ttactgaaag ttccaaagag aaggtttttt   7920
   taggctaaga taatggggct ctttacattt ccacaacata taagtaagat tagatatgga   7980
   tatgtatatg gatatgtata tggtggtaat gccatgtaat atgattatta aacttctttg   8040
   cgtccatcca aaaaaaaagt aagaattttt gaaaattcga attcg atg gct gca tca   8097
                                                     Met Ala Ala Ser
                                                     1
   gag ttc aaa gag acc ccc gaa ctc gag agt gcc gtc aga gca atg gaa     8145
   Glu Phe Lys Glu Thr Pro Glu Leu Glu Ser Ala Val Arg Ala Met Glu
   5                   10                  15                  20
   gca gca gcc aac gtg gac cca cta ttc caa tct gca ctc agt gtg ttc     8193
   Ala Ala Ala Asn Val Asp Pro Leu Phe Gln Ser Ala Leu Ser Val Phe
                   25                  30                  35
   atg tgg ctg gaa gag aat ggg att gtg act gac atg gcc aac ttc gca     8241
   Met Trp Leu Glu Glu Asn Gly Ile Val Thr Asp Met Ala Asn Phe Ala
               40                  45                  50
   ctc agc gac ccg aac gcc cat cgg atg cga aat ttt ctt gca aac gca      8289
   Leu Ser Asp Pro Asn Ala His Arg Met Arg Asn Phe Leu Ala Asn Ala
           55                  60                  65
   cca caa gca ggc agc aag tcg caa agg gcc aag tac ggg aca gca ggc      8337
   Pro Gln Ala Gly Ser Lys Ser Gln Arg Ala Lys Tyr Gly Thr Ala Gly
       70                  75                  80
   tac gga gtg gag gct cgg ggc ccc aca cca gag gaa gca cag agg gaa      8385
   Tyr Gly Val Glu Ala Arg Gly Pro Thr Pro Glu Glu Ala Gln Arg Glu
   85                  90                  95                  100
   aaa gac aca cgg atc tca aag aag atg gag acc atg ggc atc tac ttt      8433
   Lys Asp Thr Arg Ile Ser Lys Lys Met Glu Thr Met Gly Ile Tyr Phe
                   105                 110                 115
   gca aca cca gaa tgg gta gca ctc aat ggg cac cga ggg cca agc cca      8481
   Ala Thr Pro Glu Trp Val Ala Leu Asn Gly His Arg Gly Pro Ser Pro
               120                 125                 130
   ggc cag gta aag tac tgg cag aac aaa cga gaa ata ccg gac cca aac      8529
   Gly Gln Val Lys Tyr Trp Gln Asn Lys Arg Glu Ile Pro Asp Pro Asn
           135                 140                 145
   gag gac tat cta gac tac gtg cat gca gag aag agc cgg ttg gca tca      8577
   Glu Asp Tyr Leu Asp Tyr Val His Ala Glu Lys Ser Arg Leu Ala Ser
       150                 155                 160
   gaa gaa caa atc cta agg gca gct acg tcg atc tac ggg gct cca gga      8625
   Glu Glu Gln Ile Leu Arg Ala Ala Thr Ser Ile Tyr Gly Ala Pro Gly
   165                 170                 175                 180
   cag gca gag cca ccc caa gct ttc ata gac gaa gtt gcc aaa gtc tat      8673
   Gln Ala Glu Pro Pro Gln Ala Phe Ile Asp Glu Val Ala Lys Val Tyr
                   185                 190                 195
   gaa atc aac cat gga cgt ggc cca aac caa gaa cag atg aaa gat ctg      8721
   Glu Ile Asn His Gly Arg Gly Pro Asn Gln Glu Gln Met Lys Asp Leu
               200                 205                 210
   ctc ttg act gcg atg gag atg aag cat cgc aat ccc agg cgg gct cta      8769
   Leu Leu Thr Ala Met Glu Met Lys His Arg Asn Pro Arg Arg Ala Leu
           215                 220                 225
   cca aag ccc aag cca aaa ccc aat gct cca aca cag aga ccc cct ggt      8817
   Pro Lys Pro Lys Pro Lys Pro Asn Ala Pro Thr Gln Arg Pro Pro Gly
       230                 235                 240
   cgg ctg ggc cgc tgg atc agg acc gtc tct gat gag gac ctt gag gga      8865
   Arg Leu Gly Arg Trp Ile Arg Thr Val Ser Asp Glu Asp Leu Glu Gly
   245                 250                 255                 260
   tcc atc gcc acc atg gtg agc aag ggc gag gag ctg ttc acc ggg gtg      8913
   Ser Ile Ala Thr Met Val Ser Lys Gly Glu Glu Leu Phe Thr Gly Val
                   265                 270                 275
   gtg ccc atc ctg gtc gag ctg gac ggc gac gta aac ggc cac aag ttc      8961
   Val Pro Ile Leu Val Glu Leu Asp Gly Asp Val Asn Gly His Lys Phe
               280                 285                 290
   agc gtg tcc ggc gag ggc gag ggc gat gcc acc tac ggc aag ctg acc      9009
   Ser Val Ser Gly Glu Gly Glu Gly Asp Ala Thr Tyr Gly Lys Leu Thr
           295                 300                 305
   ctg aag ttc atc tgc acc acc ggc aag ctg ccc gtg ccc tgg ccc acc      9057
   Leu Lys Phe Ile Cys Thr Thr Gly Lys Leu Pro Val Pro Trp Pro Thr
       310                 315                 320
   ctc gtg acc acc ctg acc tac ggc gtg cag tgc ttc agc cgc tac ccc      9105
   Leu Val Thr Thr Leu Thr Tyr Gly Val Gln Cys Phe Ser Arg Tyr Pro
   325                 330                 335                 340
   gac cac atg aag cag cac gac ttc ttc aag tcc gcc atg ccc gaa ggc      9153
   Asp His Met Lys Gln His Asp Phe Phe Lys Ser Ala Met Pro Glu Gly
                   345                 350                 355
   tac gtc cag gag cgc acc atc ttc ttc aag gac gac ggc aac tac aag      9201
   Tyr Val Gln Glu Arg Thr Ile Phe Phe Lys Asp Asp Gly Asn Tyr Lys
               360                 365                 370
   acc cgc gcc gag gtg aag ttc gag ggc gac acc ctg gtg aac cgc atc      9249
   Thr Arg Ala Glu Val Lys Phe Glu Gly Asp Thr Leu Val Asn Arg Ile
           375                 380                 385
   gag ctg aag ggc atc gac ttc aag gag gac ggc aac atc ctg ggg cac      9297
   Glu Leu Lys Gly Ile Asp Phe Lys Glu Asp Gly Asn Ile Leu Gly His
       390                 395                 400
   aag ctg gag tac aac tac aac agc cac aac gtc tat atc atg gcc gac      9345
   Lys Leu Glu Tyr Asn Tyr Asn Ser His Asn Val Tyr Ile Met Ala Asp
   405                 410                 415                 420
   aag cag aag aac ggc atc aag gtg aac ttc aag atc cgc cac aac atc      9393
   Lys Gln Lys Asn Gly Ile Lys Val Asn Phe Lys Ile Arg His Asn Ile
                   425                 430                 435
   gag gac ggc agc gtg cag ctc gcc gac cac tac cag cag aac acc ccc      9441
   Glu Asp Gly Ser Val Gln Leu Ala Asp His Tyr Gln Gln Asn Thr Pro
               440                 445                 450
   atc ggc gac ggc ccc gtg ctg ctg ccc gac aac cac tac ctg agc acc      9489
   Ile Gly Asp Gly Pro Val Leu Leu Pro Asp Asn His Tyr Leu Ser Thr
           455                 460                 465
   cag tcc gcc ctg agc aaa gac ccc aac gag aag cgc gat cac atg gtc      9537
   Gln Ser Ala Leu Ser Lys Asp Pro Asn Glu Lys Arg Asp His Met Val
       470                 475                 480
   ctg ctg gag ttc gtg acc gcc gcc ggg atc act ctc ggc atg gac gag      9585
   Leu Leu Glu Phe Val Thr Ala Ala Gly Ile Thr Leu Gly Met Asp Glu
   485                 490                 495                 500
   ctg tac aag taa agc                                                  9600
   Leu Tyr Lys
<210> 8
   <211> 503
   <212> PRT
   <213> Artificial sequence
<220>
   <223> pVP2-VP3-GFP protein
<400> 8
   Met Ala Ala Ser Glu Phe Lys Glu Thr Pro Glu Leu Glu Ser Ala Val
   1               5                   10                  15
   Arg Ala Met Glu Ala Ala Ala Asn Val Asp Pro Leu Phe Gln Ser Ala
               20                  25                  30
   Leu Ser Val Phe Met Trp Leu Glu Glu Asn Gly Ile Val Thr Asp Met
           35                  40                  45
   Ala Asn Phe Ala Leu Ser Asp Pro Asn Ala His Arg Met Arg Asn Phe
       50                  55                  60
   Leu Ala Asn Ala Pro Gln Ala Gly Ser Lys Ser Gln Arg Ala Lys Tyr
   65                  70                  75                  80
   Gly Thr Ala Gly Tyr Gly Val Glu Ala Arg Gly Pro Thr Pro Glu Glu
                   85                  90                  95
   Ala Gln Arg Glu Lys Asp Thr Arg Ile Ser Lys Lys Met Glu Thr Met
               100                 105                 110
   Gly Ile Tyr Phe Ala Thr Pro Glu Trp Val Ala Leu Asn Gly His Arg
           115                 120                 125
   Gly Pro Ser Pro Gly Gln Val Lys Tyr Trp Gln Asn Lys Arg Glu Ile
       130                 135                 140
   Pro Asp Pro Asn Glu Asp Tyr Leu Asp Tyr Val His Ala Glu Lys Ser
   145                 150                 155                 160
   Arg Leu Ala Ser Glu Glu Gln Ile Leu Arg Ala Ala Thr Ser Ile Tyr
                   165                 170                 175
   Gly Ala Pro Gly Gln Ala Glu Pro Pro Gln Ala Phe Ile Asp Glu Val
               180                 185                 190
   Ala Lys Val Tyr Glu Ile Asn His Gly Arg Gly Pro Asn Gln Glu Gln
           195                 200                 205
   Met Lys Asp Leu Leu Leu Thr Ala Met Glu Met Lys His Arg Asn Pro
       210                 215                 220
   Arg Arg Ala Leu Pro Lys Pro Lys Pro Lys Pro Asn Ala Pro Thr Gln
   225                 230                 235                 240
   Arg Pro Pro Gly Arg Leu Gly Arg Trp Ile Arg Thr Val Ser Asp Glu
                   245                 250                 255
   Asp Leu Glu Gly Ser Ile Ala Thr Met Val Ser Lys Gly Glu Glu Leu
               260                 265                 270
   Phe Thr Gly Val Val Pro Ile Leu Val Glu Leu Asp Gly Asp Val Asn
   275 280 285
   Gly His Lys Phe Ser Val Ser Gly Glu Gly Glu Gly Asp Ala Thr Tyr
   290 295 300
   Gly Lys Leu Thr Leu Lys Phe Ile Cys Thr Thr Gly Lys Leu Pro Val
   305 310 315 320
   Pro Trp Pro Thr Leu Val Thr Thr Leu Thr Tyr Gly Val Gln Cys Phe
   325 330 335
   Ser Arg Tyr Pro Asp His Met Lys Gln His Asp Phe Phe Lys Ser Ala
   340 345 350
   Met Pro Glu Gly Tyr Val Gln Glu Arg Thr Ile Phe Phe Lys Asp Asp
   355 360 365
   Gly Asn Tyr Lys Thr Arg Ala Glu Val Lys Phe Glu Gly Asp Thr Leu
   370 375 380
   Val Asn Arg Ile Glu Leu Lys Gly Ile Asp Phe Lys Glu Asp Gly Asn
   385 390 395 400
   Ile Leu Gly His Lys Leu Glu Tyr Asn Tyr Asn Ser His Asn Val Tyr
   405 410 415
   Ile Met Ala Asp Lys Gln Lys Asn Gly Ile Lys Val Asn Phe Lys Ile
   420 425 430
   Arg His Asn Ile Glu Asp Gly Ser Val Gln Leu Ala Asp His Tyr Gln
   435 440 445
   Gln Asn Thr Pro Ile Gly Asp Gly Pro Val Leu Leu Pro Asp Asn His
   450 455 460
   Tyr Leu Ser Thr Gln Ser Ala Leu Ser Lys Asp Pro Asn Glu Lys Arg
   465 470 475 480
   Asp His Met Val Leu Leu Glu Phe Val Thr Ala Ala Gly Ile Thr Leu
   485 490 495
   Gly Met Asp Glu Leu Tyr Lys
   500
<210> 9
   <211> 33
   <212> DNA
   <213> Artificial sequence
<220> Synthetic DNA
   <223> Oligo V primer
<400> 9
   gcgcgaattc gatggcatca gagttcaaag aga 33
<210> 10
   <211> 32
<212> DNA
   <213> Artificial sequence
<220> Synthetic DNA
   <223> Oligo VI primer
<400> 10
   cgcggatccc tcaaggtcct catcagagac gg 32
<210> 11
   <211> 36
   <212> DNA
   <213> Artificial sequence
<220>
   <223> Oligo CD8 A primer
<400> 11
   aacgaggaca gttatgtccc aagcgcagaa caaata 36
<210> 12
   <211> 36
   <212> DNA
   <213> Artificial sequence
<220>
   <223> Oligo CD8 B primer
<400> 12
   tatttgttct gcgcttggga cataactgtc ctcgtt 36
<210> 13
   <211> 5676
   <212> DNA
   <213> Artificial sequence
<220>
   <223> Plasmid pFB/his-CD8-VP3
<220>
   <221> promoter
   <222> (1)..(129)
   <223> Polyhedrin promoter
<220>
   <221> CDS
   <222> (147)..(1043)
   <223> His-CD8-VP3 ORF
<220>
   <221> CDS
   <222> (222)..(257)
   <223> His-CD8 ORF
<400> 13
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcgg atctcggtcc gaaacc atg tcg tac tac cat cac cat cac cat     173
                                Met Ser Tyr Tyr His His His His His
                                1               5
   cac gat tac gat atc cca acg acc gaa aac ctg tat ttt cag ggc gcg      221
   His Asp Tyr Asp Ile Pro Thr Thr Glu Asn Leu Tyr Phe Gln Gly Ala
   10                  15                  20                  25
   aac gag gac agt tat gtc cca agc gca gaa caa ata gcc gcc atg gct      269
   Asn Glu Asp Ser Tyr Val Pro Ser Ala Glu Gln Ile Ala Ala Met Ala
                   30                  35                  40
   gca tca gag ttc aaa gag acc ccc gaa ctc gag agt gcc gtc aga gca      317
   Ala Ser Glu Phe Lys Glu Thr Pro Glu Leu Glu Ser Ala Val Arg Ala
               45                  50                  55
   atg gaa gca gca gcc aac gtg gac cca cta ttc caa tct gca ctc agt      365
   Met Glu Ala Ala Ala Asn Val Asp Pro Leu Phe Gln Ser Ala Leu Ser
           60                  65                  70
   gtg ttc atg tgg ctg gaa gag aat ggg att gtg act gac atg gcc aac      413
   Val Phe Met Trp Leu Glu Glu Asn Gly Ile Val Thr Asp Met Ala Asn
       75                  80                  85
   ttc gca ctc agc gac ccg aac gcc cat cgg atg cga aat ttt ctt gca      461
   Phe Ala Leu Ser Asp Pro Asn Ala His Arg Met Arg Asn Phe Leu Ala
   90                  95                  100                 105
   aac gca cca caa gca ggc agc aag tcg caa agg gcc aag tac ggg aca      509
   Asn Ala Pro Gln Ala Gly Ser Lys Ser Gln Arg Ala Lys Tyr Gly Thr
                   110                 115                 120
   gca ggc tac gga gtg gag gct cgg ggc ccc aca cca gag gaa gca cag      557
   Ala Gly Tyr Gly Val Glu Ala Arg Gly Pro Thr Pro Glu Glu Ala Gln
               125                 130                 135
   agg gaa aaa gac aca cgg atc tca aag aag atg gag acc atg ggc atc      605
   Arg Glu Lys Asp Thr Arg Ile Ser Lys Lys Met Glu Thr Met Gly Ile
           140                 145                 150
   tac ttt gca aca cca gaa tgg gta gca ctc aat ggg cac cga ggg cca      653
   Tyr Phe Ala Thr Pro Glu Trp Val Ala Leu Asn Gly His Arg Gly Pro
       155                 160                 165
   agc cca ggc cag gta aag tac tgg cag aac aaa cga gaa ata ccg gac      701
   Ser Pro Gly Gln Val Lys Tyr Trp Gln Asn Lys Arg Glu Ile Pro Asp
   170                 175                 180                 185
   cca aac gag gac tat cta gac tac gtg cat gca gag aag agc cgg ttg      749
   Pro Asn Glu Asp Tyr Leu Asp Tyr Val His Ala Glu Lys Ser Arg Leu
                   190                 195                 200
   gca tca gaa gaa caa atc cta agg gca gct acg tcg atc tac ggg gct      797
   Ala Ser Glu Glu Gln Ile Leu Arg Ala Ala Thr Ser Ile Tyr Gly Ala
               205                 210                 215
   cca gga cag gca gag cca ccc caa gct ttc ata gac gaa gtt gcc aaa      845
   Pro Gly Gln Ala Glu Pro Pro Gln Ala Phe Ile Asp Glu Val Ala Lys
           220                 225                 230
   gtc tat gaa atc aac cat gga cgt ggc cca aac caa gaa cag atg aaa      893
   Val Tyr Glu Ile Asn His Gly Arg Gly Pro Asn Gln Glu Gln Met Lys
       235                 240                 245
   gat ctg ctc ttg act gcg atg gag atg aag cat cgc aat ccc agg cgg      941
   Asp Leu Leu Leu Thr Ala Met Glu Met Lys His Arg Asn Pro Arg Arg
   250                 255                 260                 265
   gct cta cca aag ccc aag cca aaa ccc aat gct cca aca cag aga ccc      989
   Ala Leu Pro Lys Pro Lys Pro Lys Pro Asn Ala Pro Thr Gln Arg Pro
                   270                 275                 280
   cct ggt cgg ctg ggc cgc tgg atc agg acc gtc tct gat gag gac ctt     1037
   Pro Gly Arg Leu Gly Arg Trp Ile Arg Thr Val Ser Asp Glu Asp Leu
               285                 290                 295
   gag tga ggatccggaa ttcaaaggcc tacgtcgacg agctcactag tcgcggccgc      1093
   Glu
   tttcgaatct agagcctgca gtctcgaggc atgcggtacc aagcttgtcg agaagtacta   1153
   gaggatcata atcagccata ccacatttgt agaggtttta cttgctttaa aaaacctccc   1213
   acacctcccc ctgaacctga aacataaaat gaatgcaatt gttgttgtta acttgtttat   1273
   tgcagcttat aatggttaca aataaagcaa tagcatcaca aatttcacaa ataaagcatt   1333
   tttttcactg cattctagtt gtggtttgtc caaactcatc aatgtatctt atcatgtctg   1393
   gatctgatca ctagatctgc ctaggagatc cgaaccagat aagtgaaatc tagttccaaa   1453
   ctattttgtc atttttaatt ttcgtattag cttacgacgc tacacccagt tcccatctat   1513
   tttgtcactc ttccctaaat aatccttaaa aactccattt ccacccctcc cagttcccaa   1573
   ctattttgtc cgcccacagc ggggcatttt tcttcctgtt atgtttttaa tcaaacatcc   1633
   tgccaactcc atgtgacaaa ccgtcatctt cggctacttt ttctctgtca cagaatgaaa   1693
   atttttctgt catctcttcg ttattaatgt ttgtaattga ctgaatatca acgcttattt   1753
   gcagcctgaa tggcgaatgg gacgcgccct gtagcggcgc attaagcgcg gcgggtgtgg   1813
   tggttacgcg cagcgtgacc gctacacttg ccagcgccct agcgcccgct cctttcgctt   1873
   tcttcccttc ctttctcgcc acgttcgccg gctttccccg tcaagctcta aatcgggggc   1933
   tccctttagg gttccgattt agtgctttac ggcacctcga ccccaaaaaa cttgattagg   1993
   gtgatggttc acgtagtggg ccatcgccct gatagacggt ttttcgccct ttgacgttgg   2053
   agtccacgtt ctttaatagt ggactcttgt tccaaactgg aacaacactc aaccctatct   2113
   cggtctattc ttttgattta taagggattt tgccgatttc ggcctattgg ttaaaaaatg   2173
   agctgattta acaaaaattt aacgcgaatt ttaacaaaat attaacgttt acaatttcag 2233
   gtggcacttt tcggggaaat gtgcgcggaa cccctatttg tttatttttc taaatacatt 2293
   caaatatgta tccgctcatg agacaataac cctgataaat gcttcaataa tattgaaaaa 2353
   ggaagagtat gagtattcaa catttccgtg tcgcccttat tccctttttt gcggcatttt 2413
   gccttcctgt ttttgctcac ccagaaacgc tggtgaaagt aaaagatgct gaagatcagt 2473
   tgggtgcacg agtgggttac atcgaactgg atctcaacag cggtaagatc cttgagagtt 2533
   ttcgccccga agaacgtttt ccaatgatga gcacttttaa agttctgcta tgtggcgcgg 2593
   tattatcccg tattgacgcc gggcaagagc aactcggtcg ccgcatacac tattctcaga 2653
   atgacttggt tgagtactca ccagtcacag aaaagcatct tacggatggc atgacagtaa 2713
   gagaattatg cagtgctgcc ataaccatga gtgataacac tgcggccaac ttacttctga 2773
   caacgatcgg aggaccgaag gagctaaccg cttttttgca caacatgggg gatcatgtaa 2833
   ctcgccttga tcgttgggaa ccggagctga atgaagccat accaaacgac gagcgtgaca 2893
   ccacgatgcc tgtagcaatg gcaacaacgt tgcgcaaact attaactggc gaactactta 2953
   ctctagcttc ccggcaacaa ttaatagact ggatggaggc ggataaagtt gcaggaccac 3013
   ttctgcgctc ggcccttccg gctggctggt ttattgctga taaatctgga gccggtgagc 3073
   gtgggtctcg cggtatcatt gcagcactgg ggccagatgg taagccctcc cgtatcgtag 3133
   ttatctacac gacggggagt caggcaacta tggatgaacg aaatagacag atcgctgaga 3193
   taggtgcctc actgattaag cattggtaac tgtcagacca agtttactca tatatacttt 3253
   agattgattt aaaacttcat ttttaattta aaaggatcta ggtgaagatc ctttttgata 3313
   atctcatgac caaaatccct taacgtgagt tttcgttcca ctgagcgtca gaccccgtag 3373
   aaaagatcaa aggatcttct tgagatcctt tttttctgcg cgtaatctgc tgcttgcaaa 3433
   caaaaaaacc accgctacca gcggtggttt gtttgccgga tcaagagcta ccaactcttt 3493
   ttccgaaggt aactggcttc agcagagcgc agataccaaa tactgtcctt ctagtgtagc 3553
   cgtagttagg ccaccacttc aagaactctg tagcaccgcc tacatacctc gctctgctaa 3613
   tcctgttacc agtggctgct gccagtggcg ataagtcgtg tcttaccggg ttggactcaa 3673
   gacgatagtt accggataag gcgcagcggt cgggctgaac ggggggttcg tgcacacagc 3733
   ccagcttgga gcgaacgacc tacaccgaac tgagatacct acagcgtgag cattgagaaa 3793
   gcgccacgct tcccgaaggg agaaaggcgg acaggtatcc ggtaagcggc agggtcggaa 3853
   caggagagcg cacgagggag cttccagggg gaaacgcctg gtatctttat agtcctgtcg 3913
   ggtttcgcca cctctgactt gagcgtcgat ttttgtgatg ctcgtcaggg gggcggagcc 3973
   tatggaaaaa cgccagcaac gcggcctttt tacggttcct ggccttttgc tggccttttg 4033
   ctcacatgtt ctttcctgcg ttatcccctg attctgtgga taaccgtatt accgcctttg 4093
   agtgagctga taccgctcgc cgcagccgaa cgaccgagcg cagcgagtca gtgagcgagg 4153
   aagcggaaga gcgcctgatg cggtattttc tccttacgca tctgtgcggt atttcacacc 4213
   gcagaccagc cgcgtaacct ggcaaaatcg gttacggttg agtaataaat ggatgccctg 4273
   cgtaagcggg tgtgggcgga caataaagtc ttaaactgaa caaaatagat ctaaactatg 4333
   acaataaagt cttaaactag acagaatagt tgtaaactga aatcagtcca gttatgctgt 4393
   gaaaaagcat actggacttt tgttatggct aaagcaaact cttcattttc tgaagtgcaa 4453
   attgcccgtc gtattaaaga ggggcgtggc caagggcatg gtaaagacta tattcgcggc 4513
   gttgtgacaa tttaccgaac aactccgcgg ccgggaagcc gatctcggct tgaacgaatt 4573
   gttaggtggc ggtacttggg tcgatatcaa agtgcatcac ttcttcccgt atgcccaact 4633
   ttgtatagag agccactgcg ggatcgtcac cgtaatctgc ttgcacgtag atcacataag 4693
   caccaagcgc gttggcctca tgcttgagga gattgatgag cgcggtggca atgccctgcc 4753
   tccggtgctc gccggagact gcgagatcat agatatagat ctcactacgc ggctgctcaa 4813
   acctgggcag aacgtaagcc gcgagagcgc caacaaccgc ttcttggtcg aaggcagcaa 4873
   gcgcgatgaa tgtcttacta cggagcaagt tcccgaggta atcggagtcc ggctgatgtt 4933
   gggagtaggt ggctacgtct ccgaactcac gaccgaaaag atcaagagca gcccgcatgg 4993
   atttgacttg gtcagggccg agcctacatg tgcgaatgat gcccatactt gagccaccta 5053
   actttgtttt agggcgactg ccctgctgcg taacatcgtt gctgctgcgt aacatcgttg 5113
   ctgctccata acatcaaaca tcgacccacg gcgtaacgcg cttgctgctt ggatgcccga 5173
   ggcatagact gtacaaaaaa acagtcataa caagccatga aaaccgccac tgcgccgtta 5233
   ccaccgctgc gttcggtcaa ggttctggac cagttgcgtg agcgcatacg ctacttgcat 5293
   tacagtttac gaaccgaaca ggcttatgtc aactgggttc gtgccttcat ccgtttccac 5353
   ggtgtgcgtc acccggcaac cttgggcagc agcgaagtcg aggcatttct gtcctggctg 5413
   gcgaacgagc gcaaggtttc ggtctccacg catcgtcagg cattggcggc cttgctgttc 5473
   ttctacggca aggtgctgtg cacggatctg ccctggcttc aggagatcgg aagacctcgg 5533
   ccgtcgcggc gcttgccggt ggtgctgacc ccggatgaag tggttcgcat cctcggtttt 5593
   ctggaaggcg agcatcgttt gttcgcccag gactctagct atagttctag tggttggcta 5653
   cgtatactcc ggaatattaa tag 5676
<210> 14
   <211> 298
   <212> PRT
   <213> Artificial sequence
<220>
   <223> his-CD8-VP3 protein
<400> 14
   Met Ser Tyr Tyr His His His His His His Asp Tyr Asp Ile Pro Thr
   1               5                   10                  15
   Thr Glu Asn Leu Tyr Phe Gln Gly Ala Asn Glu Asp Ser Tyr Val Pro
               20                  25                  30
   Ser Ala Glu Gln Ile Ala Ala Met Ala Ala Ser Glu Phe Lys Glu Thr
           35                  40                  45
   Pro Glu Leu Glu Ser Ala Val Arg Ala Met Glu Ala Ala Ala Asn Val
       50                  55                  60
   Asp Pro Leu Phe Gln Ser Ala Leu Ser Val Phe Met Trp Leu Glu Glu
   65                  70                  75                  80
   Asn Gly Ile Val Thr Asp Met Ala Asn Phe Ala Leu Ser Asp Pro Asn
                   85                  90                  95
   Ala His Arg Met Arg Asn Phe Leu Ala Asn Ala Pro Gln Ala Gly Ser
               100                 105                 110
   Lys Ser Gln Arg Ala Lys Tyr Gly Thr Ala Gly Tyr Gly Val Glu Ala
           115                 120                 125
   Arg Gly Pro Thr Pro Glu Glu Ala Gln Arg Glu Lys Asp Thr Arg Ile
       130                 135                 140
   Ser Lys Lys Met Glu Thr Met Gly Ile Tyr Phe Ala Thr Pro Glu Trp
   145                 150                 155                 160
   Val Ala Leu Asn Gly His Arg Gly Pro Ser Pro Gly Gln Val Lys Tyr
                   165                 170                 175
   Trp Gln Asn Lys Arg Glu Ile Pro Asp Pro Asn Glu Asp Tyr Leu Asp
               180                 185                 190
   Tyr Val His Ala Glu Lys Ser Arg Leu Ala Ser Glu Glu Gln Ile Leu
           195                 200                 205
   Arg Ala Ala Thr Ser Ile Tyr Gly Ala Pro Gly Gln Ala Glu Pro Pro
       210                 215                 220
   Gln Ala Phe Ile Asp Glu Val Ala Lys Val Tyr Glu Ile Asn His Gly
   225                 230                 235                 240
   Arg Gly Pro Asn Gln Glu Gln Met Lys Asp Leu Leu Leu Thr Ala Met
                   245                 250                 255
   Glu Met Lys His Arg Asn Pro Arg Arg Ala Leu Pro Lys Pro Lys Pro
               260                 265                 270
   Lys Pro Asn Ala Pro Thr Gln Arg Pro Pro Gly Arg Leu Gly Arg Trp
           275                 280                 285
   Ile Arg Thr Val Ser Asp Glu Asp Leu Glu
       290                 295